# EUROPEAN PATENT SPECIFICATION

(11) **EP 2 763 599 B1**
(45) Date of publication and mention of the grant of the patent: **09.03.2022**
(21) Application number: 12766562.8
(22) Date of filing: 19.09.2012
(51) Int. Cl.: A61B 17/072

(54) **SURGICAL STAPLER WITH STATIONARY STAPLE DRIVERS**
CHIRURGISCHE KLAMMERVORRICHTUNG MIT STATIONÄREN KLAMMERTREIBERN
AGRAFEUSE CHIRURGICALE AYANT DES DISPOSITIFS D'ENTRAÎNEMENT D'AGRAFE STATIONNAIRES

(30) Priority: 23.09.2011 US 201113241922
(43) Date of publication of application: 13.08.2014
(73) Proprietor: Ethicon Endo-Surgery, Inc., Cincinnati, OH 45242 (US)
(72) Inventor: SCHMID, Katherine J., Cincinnati, Ohio 45241 (US); BAXTER, Chester O. III, Loveland, Ohio 45140 (US); ARONHALT, Taylor W., Loveland, Ohio 45140 (US); YOUNG, Joseph E., Loveland, Ohio 45140 (US); SHELTON, Frederick E. IV, Hillsboro, Ohio 45133 (US)
(74) Representative: Carpmaels & Ransford LLP
(86) International application number: PCT/US2012/056095
(87) International publication number: WO 2013/043707

(56) References cited:
- EP-A2- 1 943 962
- EP-A2- 2 005 895
- EP-A2- 2 517 642
- WO-A1-96/22055
- WO-A1-2012/044853
- US-A- 3 589 589
- US-A- 4 773 420
- US-A- 5 221 036
- US-A- 5 655 698
- US-A1- 2007 034 667
- US-A1- 2008 078 808
- US-A1- 2011 084 113
- US-B1- 6 488 196

## Description

### BACKGROUND

The present invention relates to surgical instruments and to surgical cutting and stapling instruments and staple cartridges therefor that are designed to cut and staple tissue.

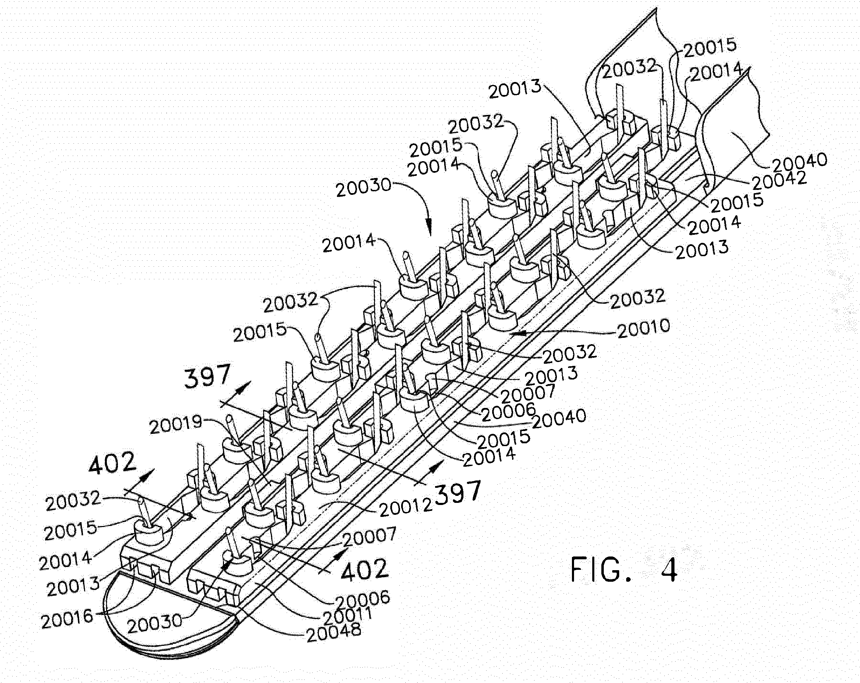

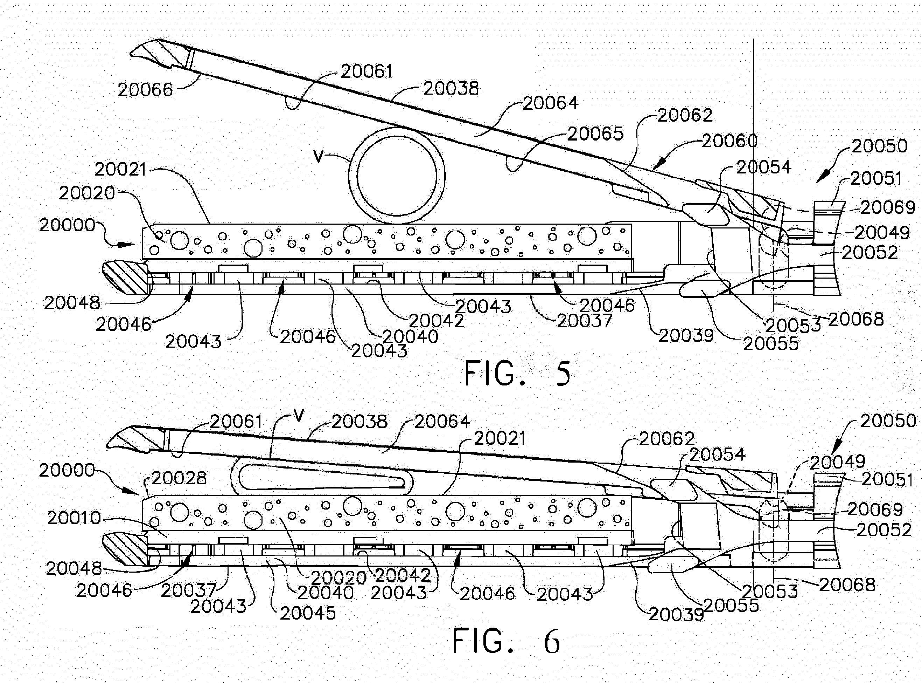

U.S. Patent No. 5,221,036 describes a device with three separate jaws. An upper jaw 10 has on its lower surface 10a a plurality of staple extruding pieces 12. A lower jaw 20 includes anvil pockets 22. An intermediate jaw 30 carries a staple cartridge 40. As a pair of handles 14, 24 is manipulated, the three jaws open and close relative to each other in predictable ways based upon the operation of linkage pins a1 - a4 and a spring 26. This is shown in figure 4 and described in column 4, lines 7 - 10 and 26 - 32 of the document. A flange 48 on the staple cartridge 40 is received by a step portion formed in the lower surface 30a of the intermediate jaw 30. This prevents the cartridge 40 from passing through a cartridge hole 32 in the intermediate jaw, as shown in figures 4 and 5, and described in column 4, lines 59 - 64 of the document. Owing to the construction of the instrument, the staple extruding pieces do not pass in to the staple slots 44 in a linear fashion, but rather arcuately. The upper jaw 10 is rotated relative to the intermediate jaw 20 so that each of the staple drivers 12 enters its respective slot 44 at an angle.

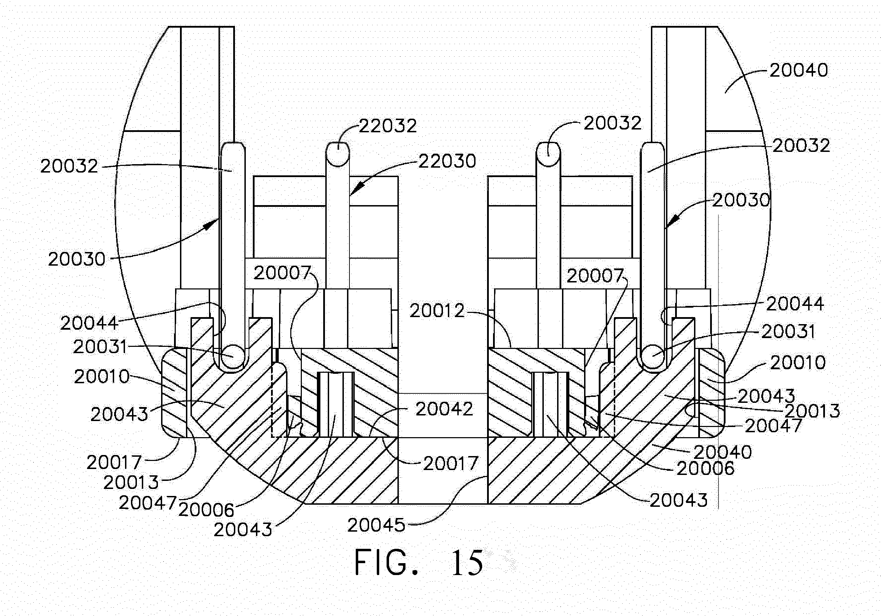

U.S. Patent No. 3,589,589 describes a parallel-jawed stapler in which a series of plates 68 that form combs 69 on a pusher head 63 form the staples against a distal anvil. As is described in column 5, line 69 to column 6, line 8 of the document, the plates 68 of the combs 69 have a rectangular cross section and correspond to the auxiliary windows of the staple slots overlapping the latter by the thickness. In order to eliminate the effect of the errors, caused by incorrect mutual disposition of the magazine slots and the plates of the pusher head, upon the interchangeability of the magazines it is advisable to provide constructive gaps 72 between the walls of the auxiliary windows 71 of the magazine slots and the plates 68 which do not lay in a common plane with the basic matched walls 66 and 67 of the magazine aperture. To provide convenient removal of the magazine 62 from the pusher head, the magazine 62 is equipped with a bevel 73 and a projection 74 on the upper lateral wall of the aperture 65. The constructive gaps 72 are shown in figure 15 of the document.

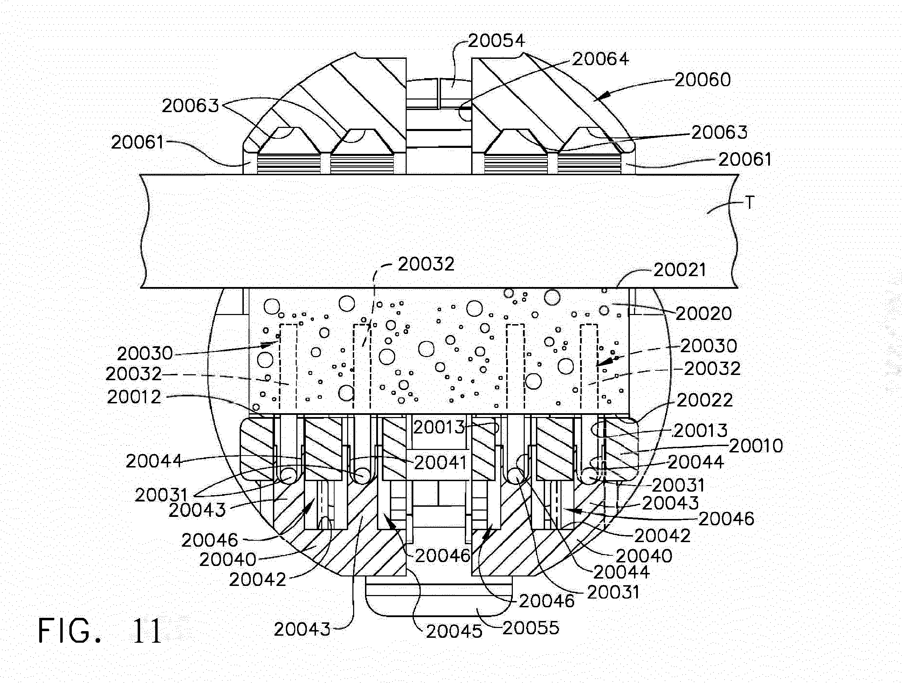

U.S. Patent No. 5,655,698 describes a specific kind of stapling device in which both jaws serve as a cartridge and an anvil, this is shown in figure 11 of the document and described in column 2, lines 56 - 62. According to column 5, lines 51 - 57 of the document, inner rows 62 and 64 of base plate protrusions are made up of staple drivers 68 configured to engage the webs 58 of staples 42 and to be received snugly within staple slots 54 of cartridge body rows 48 and 50. Similarly, outer rows 60 and 66 of the base plate protrusions are made up of anvils 69 configured to be snugly received into anvil slots 59 of cartridge body rows 46 and 52.

### SUMMARY

The present invention provides an end effector for a surgical stapling instrument comprising an staple cartridge, a support jaw and an anvil jaw. The staple cartridge comprises a deck comprising a top surface, and a plurality of staples at least partially extending through the deck, wherein each staple comprises a base and two legs extending from the base, wherein the deck is slidable relative to the staples between an unfired position and a fired position. The support jaw comprises a frame, a plurality of stationary drivers extending from the frame and immovable relative to the support jaw, wherein each stationary driver has the base of a staple positioned over its top surface such that the stationary driver is configured to support the staple when the deck is moved into the fired position, and wherein the top surface of the deck is positioned above the stationary drivers when the deck is in the unfired position, and a well defined intermediate the stationary drivers, wherein the well is configured to receive the deck when the deck is in the fired position. The anvil jaw is movably attached to the support jaw, wherein the anvil jaw is movable between an unfired position and a fired position, the anvil jaw comprising a tissue-contacting surface and a plurality of staple pockets configured to deform the staples. The anvil jaw is configured to drive the deck between its unfired position and its fired position and to deform the staples in the staple pockets as the anvil jaw is moved between its unfired position and its fired position. The deck further comprises a plurality of apertures configured to receive the stationary drivers when the deck is moved into the fired position, wherein the stationary drivers are configured to engage the sidewalls of the apertures in a press-fit manner.

### BRIEF DESCRIPTION OF THE DRAWINGS

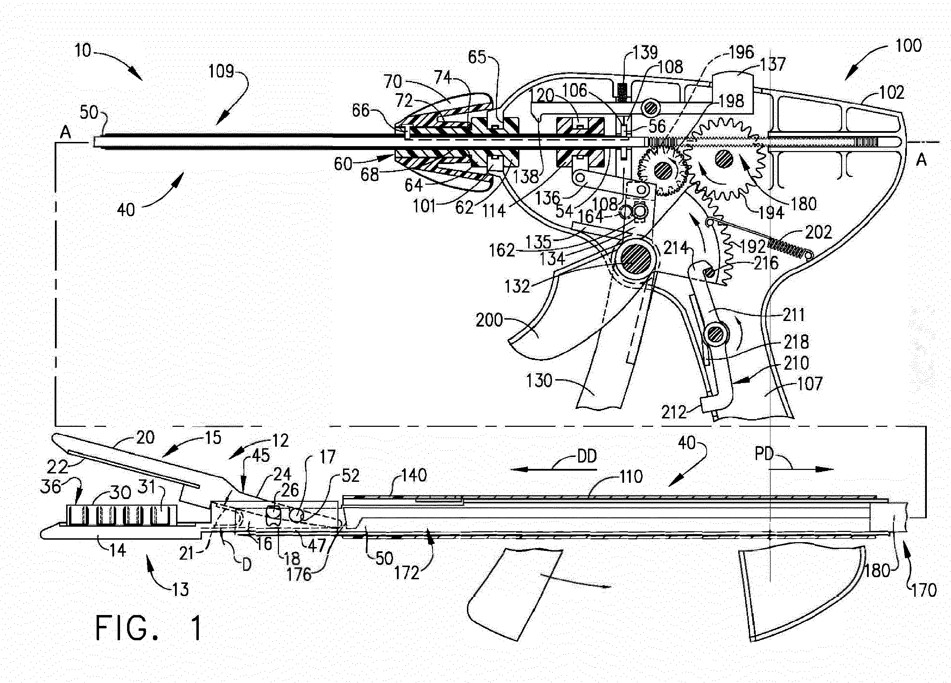

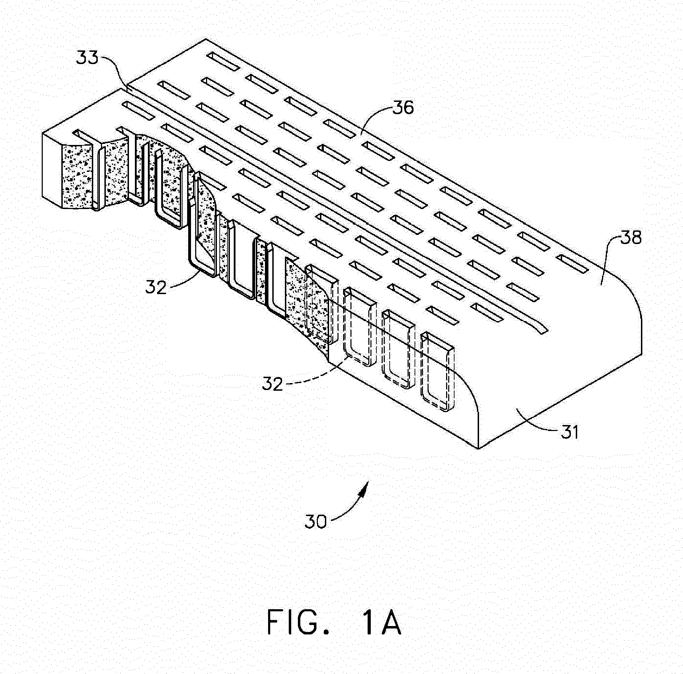

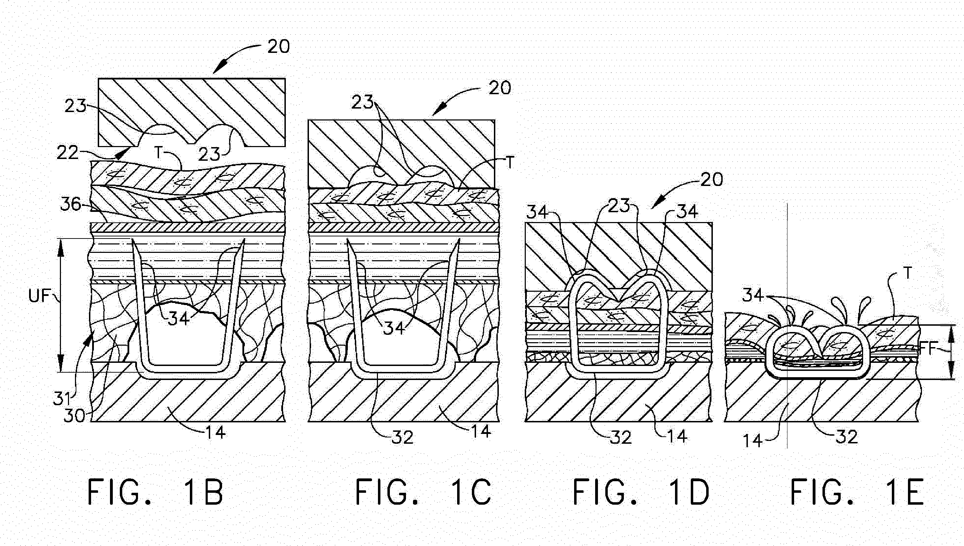

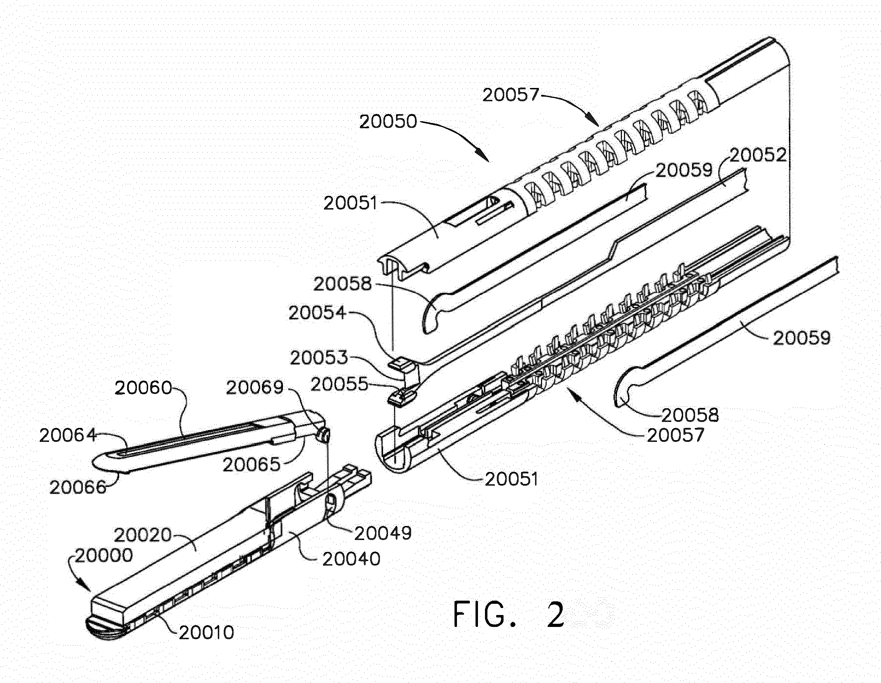

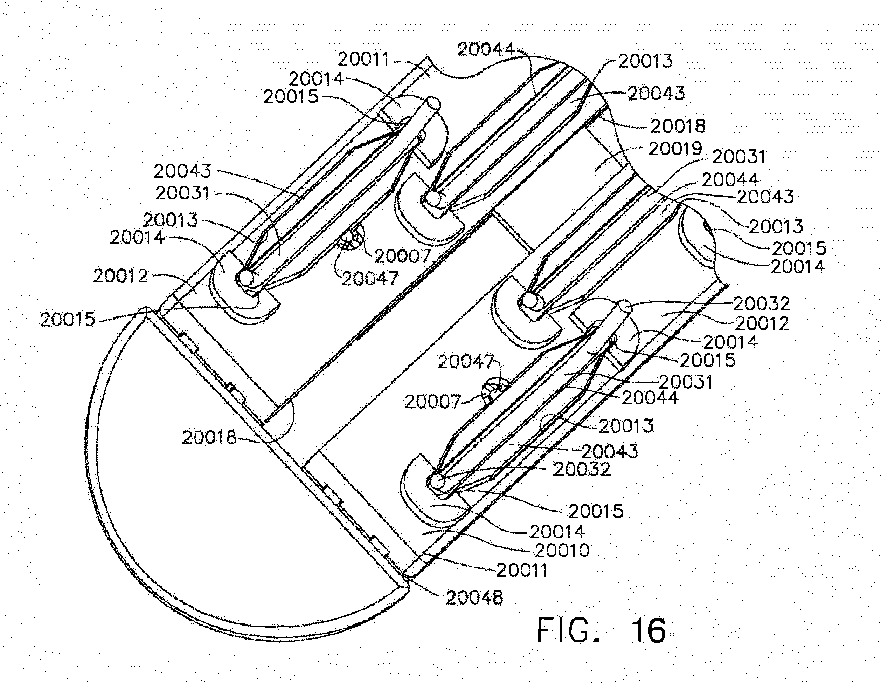

The features and advantages of this invention, and the manner of attaining them, will become more apparent and the invention itself will be better understood by reference to the following description taken in conjunction with the accompanying drawings. In the drawings. FIGS 1 - 1E illustrate the general principles of the operation of a surgical stapling instrument with an implantable staple cartridge. The end effector of this instrument is not in accordance with the invention. FIGS. 2 - 16 illustrate an end effector according to the invention for use in a surgical stapling instrument.
FIG. 1 is a cross-sectional view of a surgical instrument.
FIG. 1A is a perspective view of one implantable staple cartridge.
FIGS. 1B-1E illustrate portions of an end effector clamping and stapling tissue with an implantable staple cartridge.
FIG. 2 is an exploded view of a shaft and an end effector of a surgical stapling instrument illustrated with a staple cartridge positioned within the end effector.
FIG. 3 is an exploded view of the end effector and staple cartridge of FIG. 2.
FIG. 4 is a perspective view of a support jaw of the end effector and the staple cartridge of FIG. 2 illustrated with some components removed.
FIG. 5 is a cross-sectional elevational view of the end effector of FIG. 2 illustrated in an open configuration with a vessel positioned between the staple cartridge and an anvil jaw of the end effector.
FIG. 6 is a cross-sectional elevational view of the end effector of FIG. 2 illustrated in a partially closed configuration.
FIG. 7 is a cross-sectional elevational view of the end effector of FIG. 2 illustrated in a closed and at least partially fired configuration.
FIG. 8 is a cross-sectional elevational view of the end effector of FIG. 2 illustrated in a fully fired configuration.
FIG. 9 is a cross-sectional perspective view of the end effector of FIG. 2 illustrated in an at least partially closed, unfired configuration.
FIG. 10 is a cross-sectional perspective view of the end effector of FIG. 2 illustrated in closed, partially fired configuration.
FIG. 11 is a cross-sectional end view of the end effector of FIG. 2 illustrated in an open configuration.
FIG. 12 is a cross-sectional end view of the end effector of FIG. 2 illustrated in an at least partially closed configuration.
FIG. 13 is a cross-sectional end view of the end effector of FIG. 2 illustrated in a fired configuration.
FIG. 14 is a cross-sectional end view of the end effector of FIG. 2 illustrated in an unfired configuration with some components removed.
FIG. 15 is a cross-sectional end view of the end effector of FIG. 2 illustrated in a fired configuration with some components removed.
FIG. 16 is a top perspective view of the end effector of FIG. 2.

Corresponding reference characters indicate corresponding parts throughout the several views. The exemplifications set out herein illustrate certain embodiments of the invention, in one form, and such exemplifications are not to be construed as limiting the scope of the invention in any manner.

### DETAILED DESCRIPTION

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices are provided for performing laparoscopic and minimally invasive surgical procedures. However, the person of ordinary skill in the art will readily appreciate that the various devices disclosed herein can be used in numerous surgical procedures and applications including, for example, in connection with open surgical procedures. As the present Detailed Description proceeds, those of ordinary skill in the art will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongated shaft of a surgical instrument can be advanced.

Turning to the Drawings wherein like numerals denote like components throughout the several views, FIG. 1 depicts a surgical instrument 10 that is capable of practicing several unique benefits. The surgical stapling instrument 10 is designed to manipulate and/or actuate various forms and sizes of end effectors 12 that are operably attached thereto. In the device depicted in FIGS. 1-1E, for example, the end effector 12 includes an elongated channel 14 that forms a lower jaw 13 of the end effector 12. The elongated channel 14 is configured to support an "implantable" staple cartridge 30 and also movably support an anvil 20 that functions as an upper jaw 15 of the end effector 12.

The elongated channel 14 may be fabricated from, for example, 300 & 400 Series, 17-4 & 17-7 stainless steel, titanium, etc. and be formed with spaced side walls 16. The anvil 20 may be fabricated from, for example, 300 & 400 Series, 17-4 & 17-7 stainless steel, titanium, etc. and have a staple forming undersurface, generally labeled as 22 that has a plurality of staple forming pockets 23 formed therein. See FIGS. 1B-1E. In addition, the anvil 20 has a bifurcated ramp assembly 24 that protrudes proximally therefrom. An anvil pin 26 protrudes from each lateral side of the ramp assembly 24 to be received within a corresponding slot or opening 18 in the side walls 16 of the elongated channel 14 to facilitate its movable or pivotable attachment thereto.

In FIG. 1A, an implantable staple cartridge 30 is shown. The staple cartridge 30 has a body portion 31 that consists of a compressible hemostat material such as, for example, oxidized regenerated cellulose ("ORC") or a bio-absorbable foam in which lines of unformed metal staples 32 are supported. in order to prevent the staple from being affected and the hemostat material from being activated during the introduction and positioning process, the entire cartridge may be coated or wrapped in a biodegradable film 38 such as a polydioxanon film sold under the trademark PDS^{®} or with a Polyglycerol sebacate (PGS) film or other biodegradable films formed from PGA (Polyglycolic acid, marketed under the trade mark Vicryl), PCL (Polycaprolactone), PLA or PLLA (Polylactic acid), PHA (polyhydroxyalkanoate), PGCL (poliglecaprone 25, sold under the trademark Monocryl) or a composite of PGA, PCL, PLA, PDS that would be impermeable until ruptured. The body 31 of staple cartridge 30 is sized to be removably supported within the elongated channel 14 as shown such that each staple 32 therein is aligned with corresponding staple forming pockets 23 in the anvil when the anvil 20 is driven into forming contact with the staple cartridge 30.

In use, once the end effector 12 has been positioned adjacent the target tissue, the end effector 12 is manipulated to capture or clamp the target tissue between an upper face 36 of the staple cartridge 30 and the staple forming surface 22 of the anvil 20. The staples 32 are formed by moving the anvil 20 in a path that is substantially parallel to the elongated channel 14 to bring the staple forming surface 22 and, more particularly, the staple forming pockets 23 therein into substantially simultaneous contact with the upper face 36 of the staple cartridge 30. As the anvil 20 continues to move into the staple cartridge 30, the legs 34 of the staples 32 contact a corresponding staple forming pocket 23 in anvil 20 which serves to bend the staple legs 34 over to form the staples 32 into a "B shape". Further movement of the anvil 20 toward the elongated channel 14 will further compress and form the staples 32 to a desired final formed height "FF".

The above-described staple forming process is generally depicted in FIGS. 1B-1E. For example, FIG. 1B illustrates the end effector 12 with target tissue "T" between the anvil 20 and the upper face 36 of the implantable staple cartridge 30. FIG. 1C illustrates the initial clamping position of the anvil 20 wherein the anvil has 20 been closed onto the target tissue "T" to clamp the target tissue "T" between the anvil 20 and the upper face 36 of the staple cartridge 30. FIG. ID illustrates the initial staple formation wherein the anvil 20 has started to compress the staple cartridge 30 such that the legs 34 of the staples 32 are starting to be formed by the staple forming pockets 23 in the anvil 20. FIG. 1E illustrates the staple 32 in its final formed condition through the target tissue "T" with the anvil 20 removed for clarity purposes. Once the staples 32 have been formed and fastened to the target tissue "T", the surgeon will move the anvil 20 to the open position to enable the cartridge body 31 and the staples 32 to remain affixed to the target tissue while the end effector 12 is being withdrawn from the patient. The end effector 12 forms all of the staples simultaneously as the two jaws 13, 15 are clamped together. The remaining "crushed" body materials 31 act as both a hemostat (the ORC) and a staple line reinforcement (PGA, PDS or any of the other film compositions mentioned above 38). Also, since the staples 32 never have to leave the cartridge body 31 during forming, the likelihood of the staples 32 being malformed during forming is minimized. As used herein the term "implantable" means that, in addition to the staples, the cartridge body materials that support the staples will also remain in the patient and may eventually be absorbed by the patient's body. Such implantable staple cartridges are distinguishable from prior cartridge arrangements that remain positioned within the end effector in their entirety after they have been fired.

In various implementations, the end effector 12 is configured to be coupled to an elongated shaft assembly 40 that protrudes from a handle assembly 100. The end effector 12 (when closed) and the elongated shaft assembly 40 may have similar cross-sectional shapes and be sized to operably pass through a trocar tube or working channel in another form of access instrument. As used herein, the term "operably pass" means that the end effector and at least a portion of the elongated shaft assembly may be inserted through or passed through the channel or tube opening and can be manipulated therein as needed to complete the surgical stapling procedure. When in a closed position, the jaws 13 and 15 of the end effector 12 may provide the end effector with a roughly circular cross-sectional shape that facilitates its passage through a circular passage/opening. However, the end effectors, as well as the elongated shaft assembly, could conceivably be provided with other cross-sectional shapes that could otherwise pass through access passages and openings that have non-circular cross-sectional shapes. Thus, an overall size of a cross-section of a closed end effector will be related to the size of the passage or opening through which it is intended to pass. Thus, one end effector for example, may be referred to as a "5mm" end effector which means it can operably pass through an opening that is at least approximately 5mm in diameter.

The elongated shaft assembly 40 may have an outer diameter that is substantially the same as the outer diameter of the end effector 12 when in a closed position. For example, a 5mm end effector may be coupled to an elongated shaft assembly 40 that has 5mm cross-sectional diameter. Alternatively, a 10mm end effector may be attached to an elongated shaft that has a 5mm cross-sectional diameter. Conversely, for those applications wherein a 10mm or larger access opening or passage is provided, the elongated shaft assembly 40 may have a 10mm (or larger) cross-sectional diameter, but may also be able to actuate a 5mm or 10mm end effector. Accordingly, the outer shaft 40 may have an outer diameter that is the same as or is different from the outer diameter of a closed end effector 12 attached thereto.

As depicted, the elongated shaft assembly 40 extends distally from the handle assembly 100 in a generally straight line to define a longitudinal axis A-A. The elongated shaft assembly 40 may be approximately 9-16 inches (229-406mm) long. However, the elongated shaft assembly 40 may be provided in other lengths and, alternatively, may have joints therein or be otherwise configured to facilitate articulation of the end effector 12 relative to other portions of the shaft or handle assembly as will be discussed in further detail below. The elongated shaft assembly 40 includes a spine member 50 that extends from the handle assembly 100 to the end effector 12. The proximal end of the elongated channel 14 of the end effector 12 has a pair of retention trunnions 17 protruding therefrom that are sized to be received within corresponding trunnion openings or cradles 52 that are provided in a distal end of the spine member 50 to enable the end effector 12 to be removably coupled the elongated shaft assembly 40. The spine member 50 may be fabricated from, for example, 6061 or 7075 aluminum, stainless steel, titanium, etc.

The handle assembly 100 comprises a pistol grip-type housing that may be fabricated in two or more pieces for assembly purposes. For example, the handle assembly 100 as shown comprises a right hand case member 102 and a left hand case member (not illustrated) that are molded or otherwise fabricated from a polymer or plastic material and are designed to mate together. Such case members may be attached together by snap features, pegs and sockets molded or otherwise formed therein and/or by adhesive, screws, etc. The spine member 50 has a proximal end 54 that has a flange 56 formed thereon. The flange 56 is configured to be rotatably supported within a groove 106 formed by mating ribs 108 that protrude inwardly from each of the case members 102, 104. Such arrangement facilitates the attachment of the spine member 50 to the handle assembly 100 while enabling the spine member 50 to be rotated relative to the handle assembly 100 about the longitudinal axis A-A in a 360° path.

As can be further seen in FIG. 1, the spine member 50 passes through and is supported by a mounting bushing 60 that is rotatably affixed to the handle assembly 100. The mounting bushing 60 has a proximal flange 62 and a distal flange 64 that define a rotational groove 65 that is configured to rotatably receive a nose portion 101 of the handle assembly 100 therebetween. Such arrangement enables the mounting bushing 60 to rotate about longitudinal axis A-A relative to the handle assembly 100. The spine member 50 is non-rotatably pinned to the mounting bushing 60 by a spine pin 66. In addition, a rotation knob 70 is attached to the mounting bushing 60. For example, the rotation knob 70 has a hollow mounting flange portion 72 that is sized to receive a portion of the mounting bushing 60 therein. The rotation knob 70 may be fabricated from, for example, glass or carbon filled Nylon, polycarbonate, Ultem^{®}, etc. and is affixed to the mounting bushing 60 by the spine pin 66 as well. In addition, an inwardly protruding retention flange 74 is formed on the mounting flange portion 72 and is configured to extend into a radial groove 68 formed in the mounting bushing 60. Thus, the surgeon may rotate the spine member 50 (and the end effector 12 attached thereto) about longitudinal axis A-A in a 360° path by grasping the rotation knob 70 and rotating it relative to the handle assembly 100.

The anvil 20 is retained in an open position by an anvil spring 21 and/or another biasing arrangement. The anvil 20 is selectively movable from the open position to various closed or clamping and firing positions by a firing system, generally designated as 109. The firing system 109 includes a "firing member" 110 which, comprises a hollow firing tube 110. The hollow firing tube 110 is axially movable on the spine member 50 and thus forms the outer portion of the elongated shaft assembly 40. The firing tube 110 may be fabricated from a polymer or other suitable material and have a proximal end that is attached to a firing yoke 114 of the firing system 109. The firing yoke 114 may be over-molded to the proximal end of the firing tube 110. However, other fastener arrangements may be employed.

As can be seen in FIG. 1, the firing yoke 114 may be rotatably supported within a support collar 120 that is configured to move axially within the handle assembly 100. The support collar 120 has a pair of laterally extending fins that are sized to be slidably received within fin slots formed in the right and left hand case members. Thus, the support collar 120 may slide axially within the handle housing 100 while enabling the firing yoke 114 and firing tube 110 to rotate relative thereto about the longitudinal axis A-A. A longitudinal slot is provided through the firing tube 110 to enable the spine pin 66 to extend therethrough into the spine member 50 while facilitating the axial travel of the firing tube 110 on the spine member 50.

The firing system 109 further comprises a firing trigger 130 which serves to control the axial travel of the firing tube 110 on the spine member 50. See FIG. 1. Such axial movement in the distal direction of the firing tube 110 into firing interaction with the anvil 20 is referred to herein as "firing motion". As can be seen in FIG. 1, the firing trigger 130 is movably or pivotally coupled to the handle assembly 100 by a pivot pin 132. A torsion spring 135 is employed to bias the firing trigger 130 away from the pistol grip portion 107 of the handle assembly 100 to an un-actuated "open" or starting position. As can be seen in FIG. 1, the firing trigger 130 has an upper portion 134 that is movably attached to (pinned) firing links 136 that are movably attached to (pinned) the support collar 120. Thus, movement of the firing trigger 130 from the starting position (FIG. 1) toward an ending position adjacent the pistol grip portion 107 of the handle assembly 100 will cause the firing yoke 114 and the firing tube 110 to move in the distal direction "DD". Movement of the firing trigger 130 away from the pistol grip portion 107 of the handle assembly 100 (under the bias of the torsion spring 135) will cause the firing yoke 114 and firing tube 110 to move in the proximal direction "PD" on the spine member 50.

Different sizes and configurations of implantable staple cartridges may be employed. For example, the surgical instrument 10, when used in connection with a first firing adapter 140, may be used with a 5mm end effector 12 that is approximately 20mm long (or in other lengths) which supports an implantable staple cartridge 30. Such end effector size may be particularly well-suited, for example, to complete relatively fine dissection and vascular transactions. However, as will be discussed in further detail below, the surgical instrument 10 may also be employed, for example, in connection with other sizes of end effectors and staple cartridges by replacing the first firing adapter 140 with a second firing adapter. Alternatively, the elongated shaft assembly 40 may configured to be attached to only one form or size of end effector.

One method of removably coupling the end effector 12 to the spine member 50 will now be explained. The coupling process is commenced by inserting the retention trunnions 17 on the elongated channel 14 into the trunnion cradles 52 in the spine member 50. Thereafter, the surgeon advances the firing trigger 130 toward the pistol grip 107 of the housing assembly 100 to distally advance the firing tube 110 and the first firing adapter 140 over a proximal end portion 47 of the elongated channel 14 to thereby retain the trunnions 17 in their respective cradles 52. Such position of the first firing adapter 140 over the trunnions 17 is referred to herein as the "coupled position". An end effector locking assembly for locking the firing trigger 130 in position after an end effector 12 has been attached to the spine member 50 may be used.

More specifically, one end effector locking assembly 160 includes a retention pin 162 that is movably supported in the upper portion 134 of the firing trigger 130. As discussed above, the firing tube 110 must initially be advanced distally to the coupled position wherein the first firing adapter 140 retains the retention trunnions 17 of the end effector 12 in the trunnion cradles 52 in the spine member 50. The surgeon advances the firing adapter 140 distally to the coupled position by pulling the firing trigger 130 from the starting position toward the pistol grip 107. As the firing trigger 130 is initially actuated, the retention pin 162 is moved distally until the firing tube 110 has advanced the first firing adapter 140 to the coupled position at which point the retention pin 162 is biased into a locking cavity 164 formed in the case member. When the retention pin 162 enters into the locking cavity 164, the pin 162 may make an audible "click" or other sound, as well as provide a tactile indication to the surgeon that the end effector 12 has been "locked" onto the spine member 50. In addition, the surgeon cannot inadvertently continue to actuate the firing trigger 130 to start to form staples 32 in the end effector 12 without intentionally biasing the retention pin 162 out of the locking cavity 164. Similarly, if the surgeon releases the firing trigger 130 when in the coupled position, it is retained in that position by the retention pin 162 to prevent the firing trigger 130 from returning to the starting position and thereby releasing the end effector 12 from the spine member 50.

A firing system lock button 137 may be included that is pivotally attached to the handle assembly 100. In one form, the firing system lock button 137 has a latch 138 formed on a distal end thereof that is oriented to engage the firing yoke 114 when the firing release button is in a first latching position. As can be seen in FIG. 1, a latch spring 139 serves to bias the firing system lock button 137 to the first latching position. In various circumstances, the latch 138 serves to engage the firing yoke 114 at a point where the position of the firing yoke 114 on the spine member 50 corresponds to a point wherein the first firing adapter 140 is about to distally advance up the clamping ramp 28 on the anvil 20. It will be understood that, as the first firing adapter 140 advances axially up the clamping ramp 28, the anvil 20 will move in a path such that its staple forming surface portion 22 is substantially parallel to the upper face 36 of the staple cartridge 30.

After the end effector 12 has been coupled to the spine member 50, the staple forming process is commenced by first depressing the firing system lock button 137 to enable the firing yoke 114 to be further moved distally on the spine member 50 and ultimately compress the anvil 20 into the staple cartridge 30. After depressing the firing system lock button 137, the surgeon continues to actuate the firing trigger 130 towards the pistol grip 107 thereby driving the first staple collar 140 up the corresponding staple forming ramp 29 to force the anvil 20 into forming contact with the staples 32 in the staple cartridge 30. The firing system lock button 137 prevents the inadvertent forming of the staples 32 until the surgeon is ready to start that process. The surgeon must depress the firing system lock button 137 before the firing trigger 130 may be further actuated to begin the staple forming process.

The surgical instrument 10 may be solely used as a tissue stapling device if so desired. However, the present invention may also include a tissue cutting system, generally designated as 170. In at least one form, the tissue cutting system 170 comprises a knife member 172 that may be selectively advanced from an un-actuated position adjacent the proximal end of the end effector 12 to an actuated position by actuating a knife advancement trigger 200. The knife member 172 is movably supported within the spine member 50 and is attached or otherwise protrudes from a knife rod 180. The knife member 172 may be fabricated from, for example, 420 or 440 stainless steel with a hardness of greater than 38HRC (Rockwell Hardness C-scale) and have a tissue cutting edge 176 formed on the distal end 174 thereof and be configured to slidably extend through a slot in the anvil 20 and a centrally disposed slot 33 in the staple cartridge 30 to cut through tissue that is clamped in the end effector 12. The knife rod 180 extends through the spine member 50 and has a proximal end portion which drivingly interfaces with a knife transmission that is operably attached to the knife advance trigger 200. The knife advance trigger 200 is attached to pivot pin 132 such that it may be pivoted or otherwise actuated without actuating the firing trigger 130. A first knife gear 192 is also attached to the pivot pin 132 such that actuation of the knife advance trigger 200 also pivots the first knife gear 192. A firing return spring 202 is attached between the first knife gear 192 and the handle housing 100 to bias the knife advancement trigger 200 to a starting or un-actuated position.

The knife transmission may also include a second knife gear 194 that is rotatably supported on a second gear spindle and in meshing engagement with the first knife gear 192. The second knife gear 194 is in meshing engagement with a third knife gear 196 that is supported on a third gear spindle. Also supported on the third gear spindle 195 is a fourth knife gear 198. The fourth knife gear 198 is adapted to drivingly engage a series of annular gear teeth or rings on a proximal end of the knife rod 180. Thus, such arrangement enables the fourth knife gear 198 to axially drive the knife rod 180 in the distal direction "DD" or proximal direction "PD" while enabling the firing rod 180 to rotate about longitudinal axis A-A with respect to the fourth knife gear 198. Accordingly, the surgeon may axially advance the firing rod 180 and ultimately the knife member 172 distally by pulling the knife advancement trigger 200 towards the pistol grip 107 of the handle assembly 100.

A knife lockout system 210 may be included that prevents the advancement of the knife member 172 unless the firing trigger 130 has been pulled to the fully fired position. Such feature will therefore prevent the activation of the knife advancement system 170 unless the staples have first been fired or formed into the tissue. As can be seen in FIG. 1, various implementations of the knife lockout system 210 comprise a knife lockout bar 211 that is pivotally supported within the pistol grip portion 107 of the handle assembly 100. The knife lockout bar 211 has an activation end 212 that is adapted to be engaged by the firing trigger 130 when the firing trigger 130 is in the fully fired position. In addition, the knife lockout bar 211 has a retaining hook 214 on its other end that is adapted to hookingly engage a latch rod 216 on the first cut gear 192. A knife lock spring 218 is employed to bias the knife lockout bar 211 to a "locked" position wherein the retaining hook 214 is retained in engagement with the latch rod 216 to thereby prevent actuation of the knife advancement trigger 200 unless the firing trigger 130 is in the fully fired position.

After the staples have been "fired" (formed) into the target tissue, the surgeon may depress the firing trigger release button 167 to enable the firing trigger 130 to return to the starting position under the bias of the torsion spring 135 which enables the anvil 20 to be biased to an open position under the bias of spring 21. When in the open position, the surgeon may withdraw the end effector 12 leaving the implantable staple cartridge 30 and staples 32 behind. In applications wherein the end effector was inserted through a passage, working channel, etc. the surgeon will return the anvil 20 to the closed position by activating the firing trigger 130 to enable the end effector 12 to be withdrawn out through the passage or working channel. If, however, the surgeon desires to cut the target tissue after firing the staples, the surgeon activates the knife advancement trigger 200 in the above-described manner to drive the knife bar 172 through the target tissue to the end of the end effector. Thereafter, the surgeon may release the knife advancement trigger 200 to enable the firing return spring 202 to cause the firing transmission to return the knife bar 172 to the starting (un-actuated) position. Once the knife bar 172 has been returned to the starting position, the surgeon may open the end effector jaws 13, 15 to release the implantable cartridge 30 within the patient and then withdraw the end effector 12 from the patient. Thus, such surgical instruments facilitate the use of small implantable staple cartridges that may be inserted through relatively smaller working channels and passages, while providing the surgeon with the option to fire the staples without cutting tissue or if desired to also cut tissue after the staples have been fired.

A compressible staple cartridge that supports staples in a substantially stationary position for forming contact by the anvil is disclosed. The anvil is driven into the unformed staples wherein, for example, the degree of staple formation attained is dependent upon how far the anvil is driven into the staples. Such an arrangement provides the surgeon with the ability to adjust the amount of forming or firing pressure applied to the staples and thereby alter the final formed height of the staples.

With regard to the devices described in detail above, the amount of firing motion that is applied to the movable anvil is dependent upon the degree of actuation of the firing trigger. For example, if the surgeon desires to attain only partially formed staples, then the firing trigger is only partially depressed inward towards the pistol grip 107. To attain more staple formation, the surgeon simply compresses the firing trigger further which results in the anvil being further driven into forming contact with the staples. As used herein, the term "forming contact" means that the staple forming surface or staple forming pockets have contacted the ends of the staple legs and have started to form or bend the legs over into a formed position. The degree of staple formation refers to how far the staple legs have been folded over and ultimately relates to the forming height of the staple as referenced above. Those of ordinary skill in the art will further understand that, because the anvil 20 moves in a substantially parallel relationship with respect to the staple cartridge as the firing motions are applied thereto, the staples are formed substantially simultaneously with substantially the same formed heights.

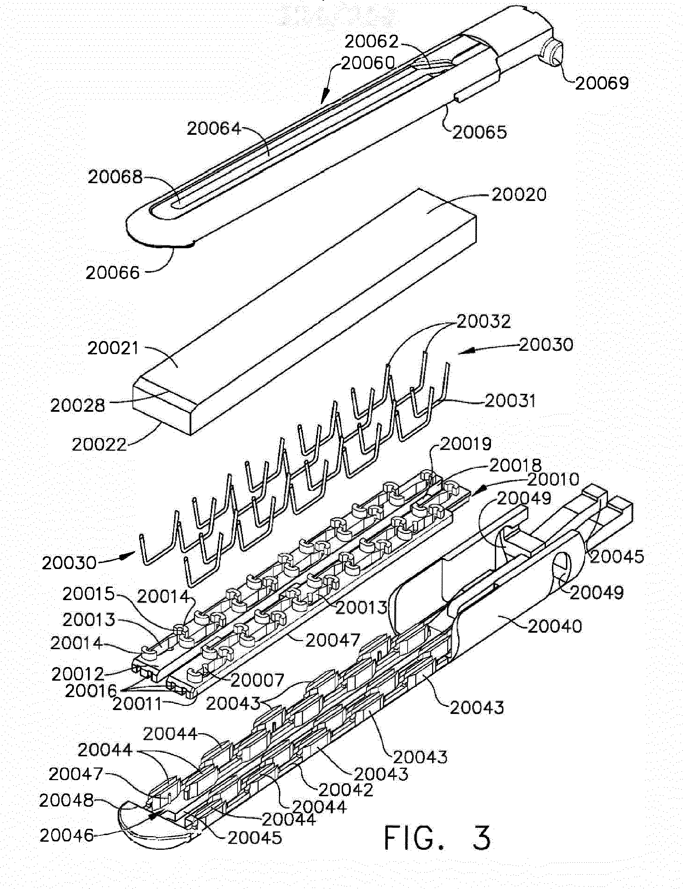

Referring now to FIG. 2, a surgical stapling instrument comprises a shaft 20050 and an end effector including a support jaw 20040 and a movable anvil jaw 20060. The surgical stapling instrument further comprises a firing system configured to advance and/or retract a firing member 20052 relative to the end effector. Exemplary firing systems are disclosed above. As illustrated in FIGS. 2 and 3, the support jaw 20040 is configured to receive and support a staple cartridge, such as staple cartridge 20000, for example, which includes a cartridge deck 20010, a plurality of staples 20030 removable retained within the deck 20010, and a tissue thickness compensator 20020. As described in greater detail further below, the firing member 20052 can be advanced distally toward the distal ends of jaws 20040 and 20060 in order to, one, move the anvil jaw 20060 into a closed position to compress tissue against the tissue thickness compensator 20020, two, deform the staples 20030, and/or, three, incise the tissue.

Referring again to FIGS. 2 and 3, the anvil 20060 comprises a proximal end 20065, a distal end 20066, and a longitudinal slot 20064 extending therebetween. Similarly, the support jaw 20040 can further comprise a longitudinal slot 20045 extending therethrough. As described in greater detail below, the slots 20064 and 20045 are configured to receive at least a portion of the firing member 20052 therein as the firing member 20052 is advanced distally and/or retracted proximally. For example, the slots can comprise closed distal ends, such as distal end 20068, for example, which can limit the travel of the firing member 20052. Referring again to FIG. 2, the shaft 20050 can comprise an articulation joint 20057 which can permit the end effector of the surgical instrument, including jaws 20040 and 20060, to articulate relative to a portion of the shaft 20050. Similar to the above, the shaft 20050 can comprise an outer housing 20051 and control arms 20059 slidably housed therein wherein each control arm 20059 can include an attachment member 20058 engaged with the support jaw 20040 of the end effector, for example, and can be configured to push and/or pull the end effector to rotate it in certain directions. Referring primarily to FIG. 4, the cartridge deck 20010, can be comprised of any suitable material, and can have any suitable geometry. In the illustrated embodiment, the cartridge deck 20010 comprises a substantially rectangular body having longitudinal channels 20016 extending therethrough.

As outlined above, the staple cartridge 20000 includes a deck 20010 and a plurality of staples 20030 at least partially contained within the deck 20010. Referring primarily to FIGS. 3 and 4, the deck 20010 includes a plurality of apertures or through holes 20013 which are each configured to receive at least a portion of a staple 20030 therein. Each staple 20030 comprises a base 20031 and two legs 20032 extending from the base 20031 in a substantially U-shaped or V-shaped configuration, for example. Each deck aperture 20013 can comprise guide slots or grooves 20015 at the proximal and distal ends thereof which can be configured to slidably receive the legs 20032 of a staple 20030 therein. As illustrated in FIG. 4, the deck 20010 can further include a top surface 20012 and a plurality of projections, or guides, 20014 extending upwardly from the top surface 20012. The guides 20014 can extend around or surround the proximal and/or distal ends of each aperture 20013. The guide slots 20015 can extend upwardly through the guides 20014. Each guide slot 20015 can be defined along an axis which is vertical. For example, each guide slot 20015 can be defined along an axis which is perpendicular to a plane defined by the top surface 20012 of the deck 20010. Alternatively, the guide slots 20015 can be defined along axes which are not vertical. For example, each guide slot 20015 can be defined along an axis which is transverse or skew to a plane defined by the top surface 20012 of the deck 20010. In either event, the legs 20032 of the staples 20030 can be resiliently biased into the guide slots 20015. In such cases, the distance between the legs 20032 in their unflexed condition can be wider than the distance between the guide slots 20015 such that the legs 20032 are resiliently flexed inwardly when the staples 20030 are positioned in the apertures 20013. For example, a tip distance can be defined between the tips of the staple legs 20032 which is wider than the width of the apertures 20013 and/or wider than the width between the guide slots 20015. In order to assemble such a staple cartridge, the legs 20032 can be biased inwardly when the staples 20030 are positioned within the apertures 20013 and, as a result of friction forces created between the staple legs 20032 and the cartridge deck 20010, the staples 20030 can be held in position.

Further to the above, the guide slots 20015 can be configured to prevent, or at least limit, relative movement between the staples 20030 and the cartridge deck 20010. The sidewalls of the guide slots can be configured such that the legs 20032 of the staples 20030 are closely received therebetween and, as a result, lateral movement between the staple legs 20032 and the cartridge deck 20010 can be prevented, or at least limited. Similarly, further to the above, relative longitudinal movement between the staple legs 20032 and the cartridge deck 20010 can be prevented, or at least limited, as the legs 20032 can be resiliently biased against proximal and distal end walls of the guide slots 20015. As a result, relative movement between the staples 20030 and the cartridge deck 20010 can be limited to movement along a deployment axis toward the anvil positioned opposite the staple cartridge 20000.

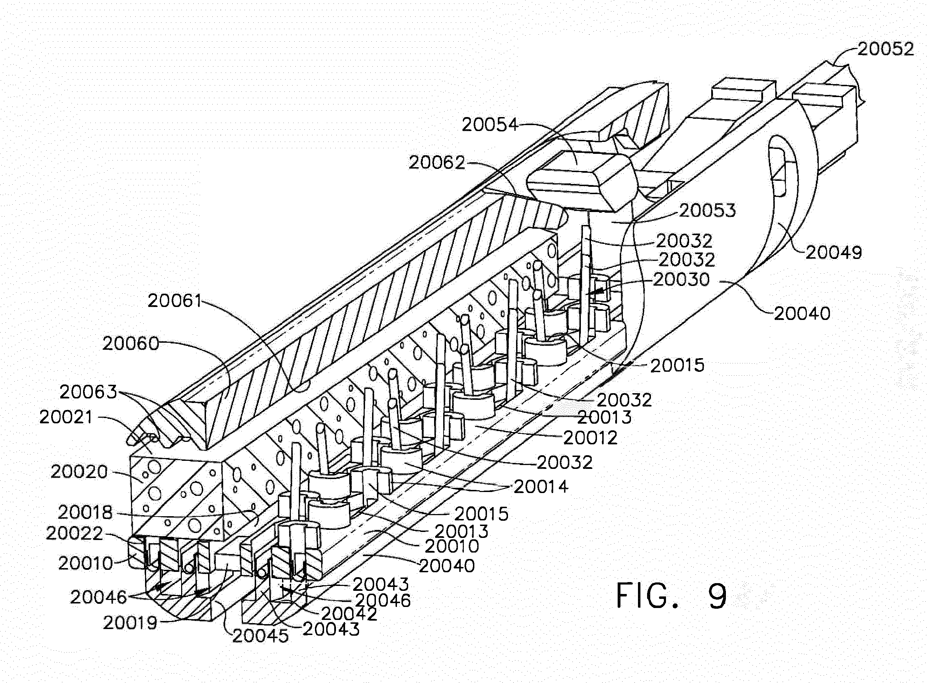

Referring again to FIG. 4, the staples 20030 can be assembled into the apertures 20013 of the deck 20010 such that the staples 20030 are removably retained in an unfired position relative to the deck 20010. When the staple cartridge 20000 is installed, inserted, and/or assembled into the support jaw 20040, referring to FIG. 2, the bases 20031 of the staples 20030 are aligned with stationary staple drivers 20043 extending upwardly from a bottom surface 20042 of the support jaw 20040, as illustrated in FIGS. 3 and 9, for example. The stationary staple drivers 20043 are immovable relative to the support jaw 20040 and can be fixed in position. The stationary staple drivers 20043 can be integrally formed with the support jaw 20040 and, the support jaw 20040 and the stationary staple drivers 20043 can be machined from a unitary piece of material, such as stainless steel or titanium, for example. The stationary drivers 20043 can be affixed or joined to the support jaw 20040 in any suitable manner, such as by welding and/or an adhesive, for example. In any event, the bases 20031 are positioned over the top surfaces of the stationary drivers 20043 such that the stationary drivers 20043 can support the staples 20030 as the staples 20030 are being deformed, as described in greater detail further below. Referring primarily to FIG. 3, each stationary driver 20043 can include a support groove or slot 20044 defined therein which can be configured to at least partially receive a base 20031 of a staple 20030 therein. The depth of the support grooves 20044 can be greater than the diameter of the staple bases 20031 positioned therein. For example, the base 20031 can be completely positioned within the support groove 20044. Alternatively, the depth of the support grooves 20044 can be less than the diameter of the staple bases 20031 positioned therein. For example, the top of each staple base 20031 can extend above the top surface of its corresponding stationary driver 20043. In any event, each support groove 20044 can comprise a contour configured to match, or at least substantially match, the contour of the staple base 20031 positioned therein. For example, the support grooves 20044 can be at least partially defined by a radius of curvature which equals, or at least substantially equals, the radius of curvature which defines the bottom of the staple bases 20031. Alternatively, the support grooves 20044 can be at least partially defined by a radius of curvature which is greater than the radius of curvature that defines the bottom of the staple bases 20031, for example. While the staples 20030 may be formed from wire having a round, or at least substantially round, outer circumference, other circumferences, such as square, oval, and/or rectangular circumferences, for example, are envisioned. In such cases, the support grooves defined in the stationary drivers 20043 can comprise square, oval, and/or rectangular profiles, respectively, configured to receive the bases of the staples.

Further to the above, the stationary drivers 20043 and the support grooves 20044 defined therein can be arranged such that they are parallel to a longitudinal slot 20018 extending through the cartridge deck 20010. In such cases, the staples 20030 can be arranged in linear, or at least substantially linear, rows, or rows in which the staples 20030 are arranged in an end-to-end manner. Alternatively, the stationary drivers 20043 and/or the support grooves 20044 can be arranged along axes which extend transversely to the longitudinal slot 20018. In such cases, the staples 20030 can be arranged in rows which are not in an end-to-end arrangement.

As illustrated in FIG. 9, which illustrates the staple cartridge 20000 in an unfired condition, the bases 20031 of the staples 20030 can be supported by the bottom surfaces of the support grooves 20044 defined within the stationary drivers 20043. In such circumstances, the weight of the staple cartridge 20000 can be supported by the stationary drivers 20043. As also illustrated in FIG. 9, the stationary drivers 20043 can extend upwardly into the apertures 20013 of the deck 20010 when the staple cartridge 20000 is in an unfired condition. In this unfired condition, the deck 20010 is positioned above a well, cavity, or depression 20046 which is defined between the stationary staple drivers 20043. The sidewalls of the apertures 20013 are in contact with the perimeter of the stationary drivers 20043, such that an interference fit is present between the stationary drivers 20043 and the sidewalls of the apertures 20013. As described in greater detail below, the deck 20010 can slide relative to the stationary drivers 20043 as the staple cartridge 20000 is being fired. As there is an interference fit between the drivers 20043 and the sidewalls of the apertures 20013, the deck 20010 slides down the stationary drivers 20043 against friction forces acting between the deck 20010 and the stationary drivers 20043. The stationary drivers 20043 can comprise an untapered profile such that each stationary driver 20043 has a constant, or at least substantially constant, outer profile, or circumference, along the height thereof. For example, the friction forces present between the deck 20010 and the stationary drivers 20043 can be constant, or at least substantially constant, as the deck 20010 is moved downwardly. Alternatively, the stationary drivers can comprise a tapered profile such that each stationary driver 20043 has an outer profile, or circumference, which changes along the height thereof. For example, the bases of the stationary drivers 20043, or the portions of the stationary drivers 20043 closest to the bottom surface 20042 of the support jaw 20040, can be wider than the tops of the stationary drivers 20043. In such cases, the friction forces present between the deck 20010 and the stationary drivers 20043 can increase as the deck 20010 is pushed downwardly toward the bottom surface 20042. In any event, the interference fit between the deck 20010 and the stationary drivers 20043, and any other portion of support jaw 20040, can hold or retain the staple cartridge 20000 in position until it is fired, as described in greater detail further below.

Referring again to FIG. 4, the legs 20032 of the staples 20030 can extend above the deck surface 20012 and/or the staple guides 20014 when the staple cartridge 20000 is in an unfired configuration. Referring to FIG. 9, the staple cartridge 20000 can further comprise a tissue thickness compensator 20020 which can be positioned adjacent to and/or against the top deck surface 20012 of the deck 20010. The legs 20032 of the staples 20030 can extend upwardly into the tissue thickness compensator 20020. For example, the tissue thickness compensator 20020 can be assembled to the staple cartridge 20000 by positioning the bottom surface 20022 of the tissue thickness compensator 20020 over the top deck surface 20012 of the deck 20010 and then inserting the staples 20030 through the openings 20013 from the bottom side 20017 of the deck 20010. For example, an adhesive can be utilized to hold the tissue thickness compensator 20020 to the cartridge deck 20010. Alternatively, the staples 20030 can be inserted into the openings 20013 and then the tissue thickness compensator 20020 can be pressed downwardly onto the staples 20030. In either event, the tips of the staple legs 20032 can be configured to penetrate the tissue thickness compensator 20020 and, the staple legs 20032 can each comprise a sharp, beveled tip, for example, which can pierce the tissue thickness compensator 20020. The reader will note that, when comparing FIGS. 4 and 9, the staple cartridge 20000 is illustrated with a tissue thickness compensator 20020 in FIG. 9 and without a tissue thickness compensator 20020 in FIG. 4. While the removal of the tissue thickness compensator 20020 in FIG. 4 has been done for the purposes of illustrating various aspects of the staple cartridge 20000, various embodiments are envisioned in which a staple cartridge could be utilized without a tissue thickness compensator. For example, the tips of the staple legs 20032 may not extend above the staple guides 20014 of the deck 20010.

Referring to FIG. 4, the support jaw 20040 can include a distal end 20048 which can be configured to provide a guide for positioning the staple cartridge 20000 within the support jaw 20040. The distal end of the cartridge deck 20010 can be aligned with the distal end 20048 and, in such a position, the bases 20031 of the staples 20030 can be aligned with the support grooves 20044. The distal end 20048 can limit the distal movement of the staple cartridge 20000. In any event, once the staple cartridge 20000 has been inserted into the support jaw 20040, referring now to FIG. 5, tissue, such as vessel V, for example, can be positioned intermediate the anvil jaw 20060 and the staple cartridge 20000. As illustrated in FIGS. 5 and 6, the anvil jaw 20060 is movable between an open position (FIG. 5) and a partially-closed position (FIG. 6) wherein, as described in greater detail further below, the surgical stapling instrument can include a firing member 20052 which can be advanced distally to engage the anvil jaw 20060 and rotate the anvil jaw 20060 toward the staple cartridge 20000 and the support jaw 20040. For example, the firing member 20052 can comprise a top cam driver 20054 and a bottom cam driver 20055 which can be configured to engage the anvil jaw 20060 and the support jaw 20040, respectively. More particularly, the firing member 20052 can be advanced from an unfired position (FIG. 5) in which the cam drivers 20054 and 20055 are not engaged with the jaws 20040 and 20060 to a partially-advanced position (FIG. 6) in which the top cam driver 20054 is in contact with a cam surface 20062 on the anvil jaw 20060 and the bottom cam driver 20055 is in contact with a cam surface 20039 on the support jaw 20040. As the firing member 20052 is progressed distally, the anvil jaw 20060 can be moved toward the support jaw 20040 thereby bringing a tissue-contacting surface 20061 into contact with the vessel V, and/or any other suitable tissue positioned between the jaws 20040 and 20060. For example, the top cam driver 20054 can slide relative to the cam surface 20062 and cam the anvil jaw 20060 toward the staple cartridge 20000 until the top cam driver 20054 reaches the top, or outer, surface 20038 of the anvil jaw 20060. Similarly, the bottom cam driver 20055 can slide relative to the cam surface 20039 until the bottom cam driver 20055 reaches the bottom, or outside, surface 20037 of the support jaw 20040. The bottom cam driver 20055 can reach the bottom surface 20037 of the support jaw 20040 before the top cam driver 20054 reaches the top surface 20038 of the anvil jaw 20060. The bottom cam driver 20055 can reach the bottom surface 20037 of the support jaw 20040 after the top cam driver 20054 reaches the top surface 20038 of the anvil jaw 20060. Alternatively, the bottom cam driver 20055 can reach the bottom surface 20037 at the same time that the top cam driver 20054 reaches the top surface 20038. In any event, once the cam drivers 20054 and 20055 have reached their respective surfaces 20038 and 20037, the anvil jaw 20060 can be in a fully closed position.

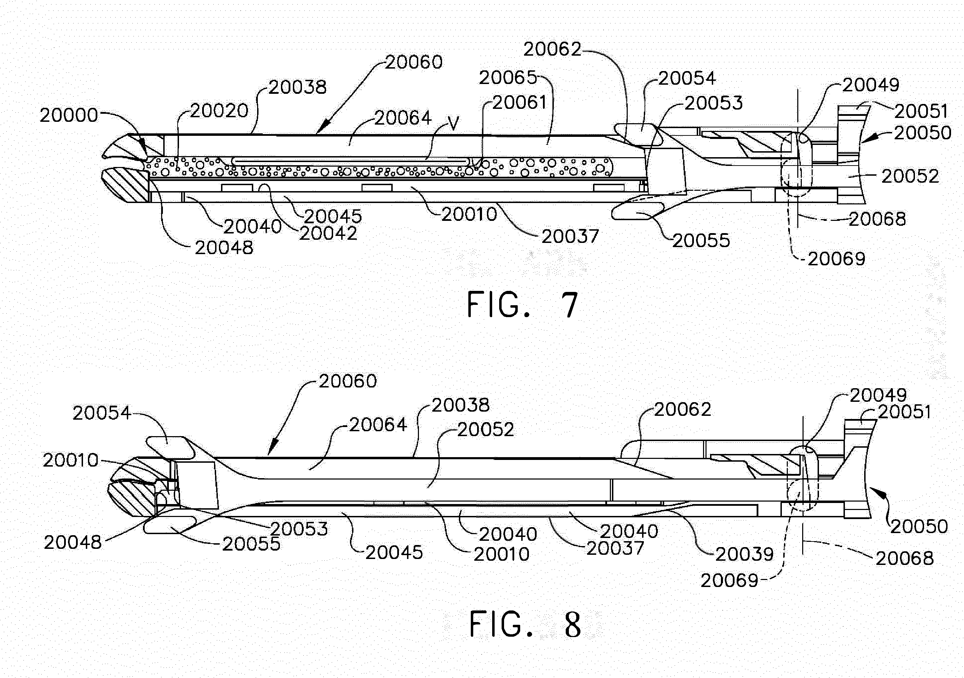

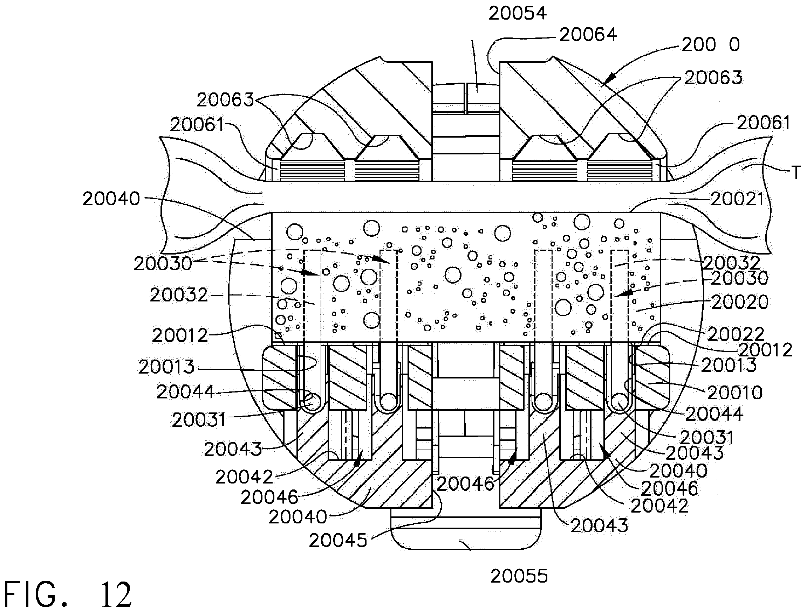

Further to the above, a surgeon can utilize the surgical stapling instrument to grasp, manipulate, and/or otherwise evaluate the tissue positioned between the jaws 20040 and 20060. For example, the surgeon may only partially advance the firing member 20052 such that the anvil 20060 is only moved to a partially-closed position, as illustrated in FIG. 6. In the event that the surgeon wishes to re-evaluate the positioning of the end effector, the surgeon may retract the firing member 20052 thereby allowing the anvil 20060 to return to an open position. The surgical instrument can further include a spring configured to bias the anvil 20060 into an open position such that the anvil jaw 20060 can return to an open position after the firing member 20052 has been retracted. In such cases, the surgeon can partially close and re-open the anvil 20060 as many times as appropriate in order to properly position the tissue between the anvil jaw 20060 and the staple cartridge 20000. FIGS. 11 and 12 depict an end view of the anvil jaw 20060 being moved between an open position and an at least partially-closed position. When the anvil jaw 20060 is in the partially-closed position illustrated in FIG. 6, the reader will note that the vessel V has distorted from the compression force applied thereto; however, the reader will also note that the tissue thickness compensator 20020 has not distorted, or at least substantially distorted, from the same compression force. The reader will also note that the tissue T in FIG. 11 can be distorted when it is compressed by an at least partially closed anvil jaw 20060, as illustrated in FIG. 12, although the tissue thickness compensator 20020 may remain undistorted. In such cases, the tissue thickness compensator 20020 can comprise a modulus of elasticity that is greater than the modulus of elasticity of the tissue T and/or the vessel V positioned between the tissue thickness compensator 20020 and the tissue contacting surface 20061 of the anvil jaw 20060. Such can allow the tissue and/or vessel to be compressed and/or manipulated without pushing the tissue or vessel against the tips of the staples 20030 embedded within the tissue thickness compensator 20020. As the anvil 20060 is pushed closer toward the support jaw 20040, as illustrated in FIG. 7, the tissue thickness compensator 20020 can compress downwardly toward the deck 20010. In such circumstances, depending on the height of the staple legs 20032, the tips of the staple legs 20032 may or may not at least partially pierce the tissue or vessel. In either event, referring now to FIG. 8, the firing member 20052 can be further advanced toward the distal end of the end effector to fire the staple cartridge 20000, as described in greater detail below.

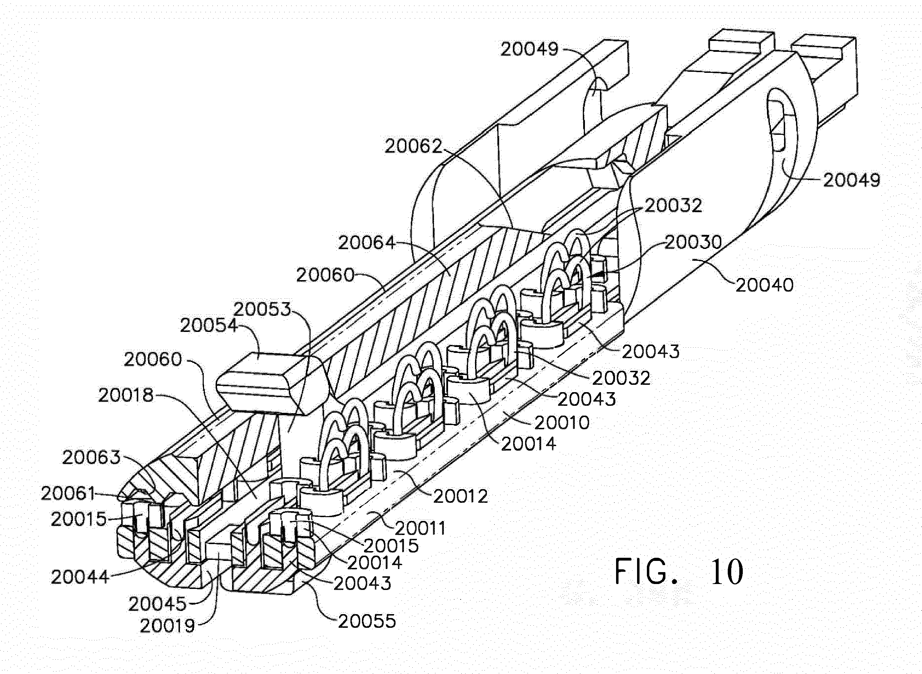

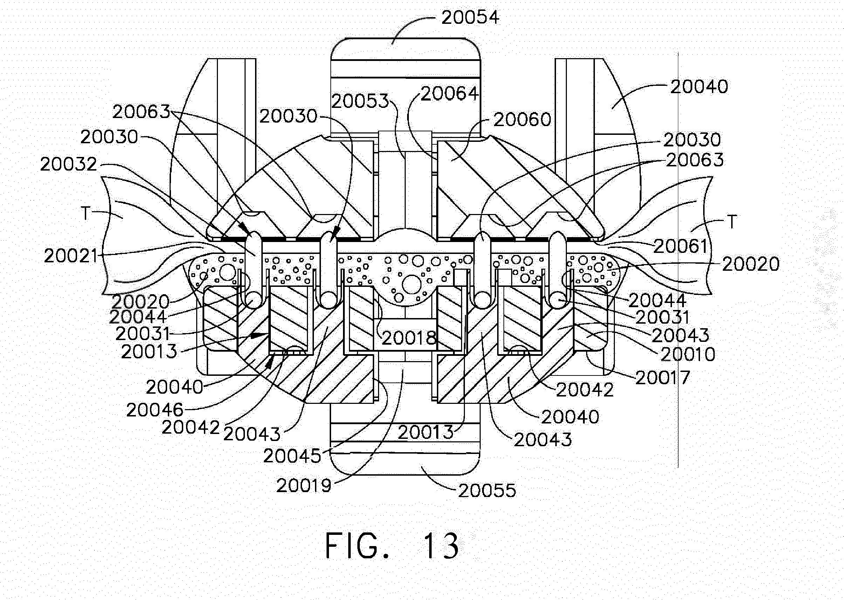

As discussed above, the cartridge deck 20010 can be slid downwardly toward the bottom surface 20042 of the support jaw 20040 as the staple cartridge 20000 is being fired. Referring to FIGS. 6 and 7, the reader will note that the cartridge deck 20010 is moved from an unfired position (FIG. 6) to a fired position (FIG. 7) as the firing member 20052 is advanced distally. The movement of the cartridge deck 20010 between an unfired position and a fired position is also illustrated in FIGS. 9 and 10 and, in addition, in FIGS. 12 and 13. Upon comparing FIGS. 9 and 10, the cartridge deck 20010 can be pushed downwardly into the well 20046 as the staples 20030 are being deformed. More specifically, as the anvil jaw 20060 is pushed downwardly toward the support jaw 20040, the tissue contacting surface 20061 contacts tissue positioned intermediate the tissue contacting surface 20061 and the top surface 20021 of the tissue thickness compensator 20020 and push the tissue downwardly toward the support jaw 20040. This compressive force also pushes the top surface 20021 of the tissue thickness compensator 20020 downwardly toward the support jaw 20040 thereby compressing the tissue thickness compensator 20020. As the tissue thickness compensator 20020 is compressed, the top surface 20021 is pushed below the tips of the staple legs 2002 such that the staple legs 20032 emerge from the tissue thickness compensator 20032 and pierce the tissue. Further downward movement of the anvil 20060 positions one or more forming surfaces on the anvil jaw 20060 against the staple legs 20032. The anvil jaw 20060 comprises a plurality of forming pockets 20063 which are configured to receive the staple legs 20032 of the staples 20030 and bend or curl the staple legs 20032 downwardly, for example. Ultimately, the anvil jaw 20060 can be pushed downwardly until the staples 20030 have been sufficiently deformed, or fired.

Further to the above, the anvil jaw 20060 can be moved toward the support jaw 20040 until the staples 20030 have been deformed, or fired, to a desired height. The firing member 20052 can be advanced distally to push the anvil 20060 downwardly until the desired deformed height of the staples 20030 has been reached. In at least one circumstance, the firing member 20052 can push the anvil jaw 20060 to its final height when the upper cam driver 20054 has reached the top surface 20038 of the anvil jaw 20060 and the bottom cam driver 20055 has reached the bottom surface 20039 of the support jaw 20040. In such circumstances, the movement of the anvil jaw 20060 to its fully-closed position is sufficient to fully form the staples 20030 to their desired deformed height. In at least one such circumstance, all of the staples 20030 can be deformed to their desired height simultaneously. In various circumstances, the anvil 20060 can be rotated between its open position and its closed position wherein the rotation of the anvil jaw 20060 can be centered about an axis defined by closure pins 20069 extending from the anvil jaw 20060. Referring primarily to FIG. 3, the closure pins 20069 can extend from the lateral sides of the anvil jaw 20060 and can be positioned along an axis of rotation. The support jaw 20040 can include pin slots 20049 defined in opposite sides thereof which are each configured to receive a pin 20069 therein. As described in greater detail below, the pins 20069 can rotate and/or translate within the pin slots 20049. When the anvil jaw 20060 is moved from an open position to a partially-closed position as illustrated in FIGS. 5 and 6, the anvil jaw 20060 can be rotated about the pins 20069 after being contacted by the advancing firing member 20052. Along these lines, the reader will note that the closure pins 20069 have remained in the top portions of the pin slots 20049 which indicates that, in the current circumstances, the anvil jaw 20060 has not yet been translated downwardly. Various other circumstances are envisioned in which the anvil jaw 20060 is translated in addition to or in lieu of being rotated as it is moved from its open position to a partially-closed position. Upon comparing FIGS. 6 and 7, the reader will note that the firing member 20052 has been advanced distally and that the anvil jaw 20060 has been moved further toward the support jaw 20040. While the anvil jaw 20060 has been rotated between its position in FIG. 6 and its position in FIG. 7, the anvil 20060 has also translated toward the support jaw 20040, as evidenced by the downward movement of the pins 20069 in the pin slots 20049.

Further to the above, the anvil jaw 20060 can float downwardly as it is closed. The anvil jaw 20060 can translate and/or rotate downwardly such that the tissue-contacting surface 20061 of the anvil jaw 20060 moves downwardly in a level, or at least substantially level, manner. When the anvil jaw 20060 is level as it is closed, the forming pockets 20063 of the anvil jaw 20060 can deform all, or at least nearly all, of the staples 20030 at the same time. Furthermore, the forming pockets 20063 can deform all, or at least nearly all, of the staples 20030 to the same, or at least substantially the same, height, as the anvil is floated downwardly. The anvil 20060 can also float as the firing member 20052 is advanced distally and/or retracted proximally. The anvil 20060 can tilt proximally and/or distally and/or otherwise adjust as the staple cartridge 20000, for example, is being fired.

As outlined above, the pins 20069 of the anvil jaw 20060 may slide downwardly within the pin slots 20049 of the support jaw 20040 when the anvil jaw 20060 is moved between its position in FIG. 6 and its position in FIG. 7. Each pin slot 20049 can be defined along a longitudinal axis 20068 and can be configured to limit the movement of the pins 20069 along a straight line, or an at least substantially straight line. The longitudinal axes 20068 can be perpendicular, or at least substantially perpendicular, to the bottom surface 20042 of the support jaw 20040. In such cases, the anvil jaw 20060 can move in a parallel, or an at least substantial parallel, path toward the bottom surface 20042 of the support jaw 20040. More particularly, the tissue-contacting surface 20061 can comprise a flat, or planar, surface which can be moved downwardly toward the support jaw 20040 such that the tissue-contacting surface 20061 remains parallel, or at least substantially parallel, to a planar surface defining the bottom surface 20042. the tissue-contacting surface 20061 can be moved downwardly toward the top surface 20021 of the tissue thickness compensator 20020 such that the tissue-contacting surface 20061 remains parallel, or at least substantially parallel, to the top surface 20021. the tissue-contacting surface 20061 can be moved downwardly toward the deck surface 20012 of the cartridge deck 20010 such that the tissue-contacting surface 20061 remains parallel, or at least substantially parallel, to the deck surface 20012. The deck 20010 can be pushed downwardly such that the bottom surface 20017 of the deck 20010 remains parallel, or at least substantially parallel, to the bottom surface 20042 of the support jaw 20040. Further to the above, the longitudinal axes 20068 can extend transversely with respect to the bottom surface 20042 of the support jaw 20040, the deck surface 20012 of the cartridge deck 20010, and/or the top surface 20021 of the tissue thickness compensator 20020, for example. The top surface 20038 of the anvil jaw 20060 can be parallel to the tissue contacting surface 20061 of the anvil jaw 20060 and, similarly, the outer surface 20038 of the support jaw 20040 can be parallel to the bottom surface 20042 of the support jaw 20040.

Further to the above, the longitudinal axes 20068 can be perpendicular, or at least substantially perpendicular, to a plane defined by the top surfaces of the stationary staple drivers 20043. In such cases, the anvil jaw 20060 can move in a parallel, or an at least substantial parallel, path toward this plane. More particularly, the tissue-contacting surface 20061 can comprise or define a flat, or planar, surface which can be moved downwardly toward the support jaw 20040 such that the tissue-contacting surface 20061 remains parallel, or at least substantially parallel, to the top surfaces of the stationary staple drivers 20043. However, not all of the top surfaces of the stationary staple drivers 20043 may lie in the same plane. The stationary staple drivers 20043 may have different heights and, thus, the top surfaces of the staple drivers 20043 may lie within different planes. The stationary staple drivers 20043 within a first row may have a first height and the stationary staple drivers 20043 within a second row may have a second height. For example, the tips of the staples 20030 supported by such stationary staple drivers 20043 can be supported at different heights with respect to the bottom surface 20042 of the support jaw 20040 in their unfired position. In such circumstances, especially where all of the staples 20030 have the same or at least substantially the same unfired height, the staples 20030 supported by taller stationary staple drivers 20043 can be deformed a greater amount than the staples 20030 supported by the shorter stationary staple drivers 20043. The staples 20030 can have different unfired heights. For example, staples 20030 having a shorter unfired height can be positioned on the taller stationary staple drivers 20043 while the staples 20030 having a taller unfired height can be positioned on the shorter stationary staple drivers 20043, for example. Alternatively, staples 20030 having a shorter unfired height can be positioned on the shorter stationary staple drivers 20043 while the staples 20030 having a taller unfired height can be positioned on the taller stationary staple drivers 20043. The support jaw 20040 can include several rows of stationary staple drivers 20043 having a first height and several rows of stationary staple drivers 20043 having a second height. The support jaw 20040 can include a first row of stationary staple drivers 20043 having a first height, a second row of stationary staple drivers 20043 having a second height, and one or more additional rows of stationary staple drivers 20043 having a height which is different than the first height and the second height. as described above, the height of a stationary staple driver 20043 can be measured between the bottom surface 20042 and the top surface of the stationary staple driver 20043; alternatively, the height of a stationary staple driver 20043 can be measured from the bottom surface 20042 to the bottom of the staple support groove 20044 defined therein.

As discussed above, the firing member 20052 can be advanced distally in order to position the anvil 20060 in a fully closed position. In some circumstances, as also discussed above, the firing member 20052 can fully close the anvil 20060 when the top camming member 20054 of the firing member 20052 first engages the top surface 20038 of the anvil jaw 20060 and the bottom camming member 20055 first engages the bottom surface 20039 of the support jaw 20040. In various circumstances, however, the initial distal movement of the firing member 20052 may not be sufficient to move the anvil 20060 to its fully closed position and fully form the staples 20030. In such circumstances, the firing member 20052 may progressively move the anvil 20060 into its fully fired position as the firing member 20052 is moved distally. The firing member 20052 can first form the proximal-most staples 20030 to their fully-fired position and then progressively form each staple 20030 to its fully deformed height as the firing member 20052 passes the staples 20030. In such cases, a fixed distance, or height, can be defined between the top camming member 20054 and the bottom camming member 20055 such that anvil jaw 20060 and the support jaw 20040 are closer together than the fixed height defined between the camming members 20054 and 20055. Thus, the height of the anvil jaw 20060 can be controlled, or at least controlled in the region of the end effector proximate the camming members 20054 and 20055. As the firing member 20052 is advanced distally, the staples 20030 of the staple cartridge 20000 can be formed to their final deformed heights until the firing member 20052 reaches the distal end of the end effector. At such point, the firing member 20052 can be retracted to its proximal, unfired position and the anvil jaw 20060 can be reopened. In some circumstances, the firing member 20052 can be retracted prior to being advanced to the distal end of the end effector.

As the cartridge deck 20010 is moved downwardly into the well 20046 from its unfired position to its fired position, referring now to FIGS. 12 and 13, the cartridge deck 20010 can become lodged within the well 20046. More particularly, as outlined above, the cartridge deck 20010 engages the support jaw 20040 in an interference-fit or press-fit manner as the cartridge deck 20010 is pushed downwardly such that the cartridge deck 20010 can be held in its fired position. In certain circumstances, the staples 20030 may not be engaged with the deck 20010 when the deck 20010 is in its fired position and, thus, when the anvil jaw 20060 is reopened after the staple cartridge 20000 has been fired, the staples 20030, the tissue T captured within the staples 20030, and/or the tissue thickness compensator 20020 can be lifted away from the support jaw 20040 and the cartridge deck 20010. For example, the cartridge deck 20010 can remain affixed to the support jaw 20040 until the cartridge deck 20010 is removed from the support jaw 20040. As discussed above, the sidewalls of the apertures 20013 defined within the deck 20010 are configured to engage the stationary staple drivers 20043 as the cartridge deck 20010 descends into the well 20046. The support jaw 20040 and the cartridge deck 20010 can include other co-operating features which create an interference fit therebetween. Such co-operating features could include apertures defined in the support jaw 20040 and downwardly-depending cones extending from the cartridge deck 20010, for example, which could be configured to enter into the apertures defined in the support jaw 20040 and engage the sidewalls thereof. The cartridge deck 20010 can comprise a unitary piece of material which moves downwardly as the staple cartridge 20000 is being fired. Alternatively, the cartridge deck 20010 can comprise several portions which can move relative to one another. For example, the portions of the cartridge deck 20010 can be held together by one or more frangible connectors which can be configured to break as the staple cartridge 20000 is being compressed by the anvil jaw 20060. The connectors can be configured to hold the portions of the cartridge deck 20010 together when the anvil jaw 20060 is closed but break as the firing member 20052 passes thereby. In such cases, the connected portions of the cartridge deck 20010 can be progressively released from the cartridge deck 20010 as the firing member 20052 is moved distally. Thus, the proximal-most portions of the cartridge deck 20010 can be pushed downwardly to their fully-fired positions before the middle portions and the distal portions of cartridge deck 20010 are pushed downwardly to their fully-fired positions. As the firing member 20052 is advanced, the middle portions of the cartridge deck 20010 can be pushed downwardly to their fully-fired positions followed by the distal-most portions of the cartridge deck 20010, for example.

Further to the above, the firing member 20052 can further comprise a cutting portion 20053, such as a knife edge, for example, which can be configured to incise the tissue as the firing member is progressed from its unfired position to a fired position. The cutting portion 20053 can be positioned and arranged such that it lags the cam drivers 20054 and 20055 and passes through the longitudinal slot 20018. In such cases, the cam drivers 20054 and 20055 can progressively deform the staples 20030 to the desired height, or at least assure that staples 20030 have been deformed to the desired height, as the cutting portion 20053 follows behind the drivers 20054 and 20055 and progressively cuts the tissue. Referring again to FIG. 4, the cartridge deck 20010 can comprise a plurality of portions, such as first and second sides, for example, which can be held together by one or more cuttable ties holding the plurality of portions together. For example, the cartridge deck 20010 can comprise ties 20019 positioned within the slot 20018 which can be severed and/or broken by the cutting portion 20053 as the cutting portion 20053 is moved from its unfired position to its fired position. Thus, the first and second portions of the cartridge deck 20010 can be progressively released from one another as the firing member 20052 is advanced. The ties 20019 may be sufficiently frangible such that they can break as the cartridge deck 20010 is pushed downwardly by the anvil jaw 20060 to deform the staples 20030, for example. For example, one or more of the ties 20019 may already be broken prior to the advancement of the firing member 20052.

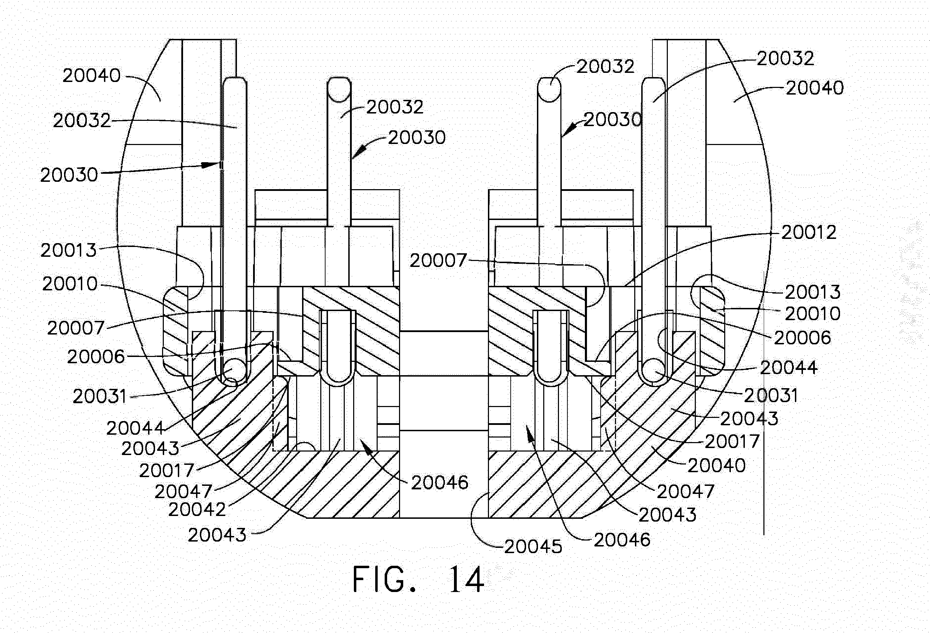

As described above, the staple cartridge 20000 can be supported on the support jaw 20040 in any suitable manner. Referring now to FIG. 3, the support jaw 20040 can further comprise supports 20047 which can be configured to support the cartridge deck 20010. For example, the supports 20047 can extend vertically along the sides of the stationary staple drivers 20043 and can each comprise a top surface against which the cartridge deck 20010 can be positioned. Referring now to FIG. 4, the cartridge deck 20010 can include ledges 20006 which can abut the supports 20047. For example, each ledge 20006 can at least partially define the end of a channel 20007 extending through the body 20011 of the cartridge deck 20010. Referring now to FIG. 14, each ledge 20006 can be defined by a certain thickness extending between a bottom surface 20017 of the ledge 20006 to a top surface wherein the bottom surface 20017 can contact a top portion of a support 20047. The bottom surfaces 20017 of the ledges 20006 may not contact the supports 20047 in the unfired condition of the staple cartridge 20000; however, in such cases, the bottom surfaces 20017 can come into contact with the supports 20047 as the staple cartridge 20000 is fired and the cartridge deck 20010 is moved downwardly. When the ledges 20006 of the cartridge deck 20010 are in contact with the supports 20047, the downward movement of the cartridge deck 20010 can be prevented, or at least inhibited, until the ledges 20006 deform and/or break thereby allowing the cartridge deck 20010 to move downwardly into its fired position. Referring to FIG. 15, the ledges 20006 can be configured to break, fold, and/or otherwise deflect inwardly into the channels 20007. When the ledges 20006 have been folded into the channels 20007, sufficient clearance can exist between the cartridge deck 20010 and the stationary staple drivers 20043 to permit the cartridge deck 20010 to move relative thereto. Further to the above, the ledges 20006 can break, snap, and/or otherwise plastically deform, in order to permit relative movement between the cartridge deck 20010 and the support jaw 20040. The ledges 20006 may not need to break in order to permit such relative movement. For example, the ledges 20006 can be configured to flex, bend, and/or otherwise elastically deform, into the channels 20007, for example, to permit relative movement between the cartridge deck 20010 and the support jaw 20040.

Further to the above, regardless of whether the ledges 20060, and/or any other suitable support members, of the cartridge deck 20010 plastically deform and/or elastically deform, the co-operation of the ledges 20006 and the supports 20047 can prevent, or at least inhibit, the downward movement of the cartridge deck 20010 until a certain force or pressure has been applied to the staple cartridge 20000 by the anvil jaw 20060 and/or the firing member 20052. The ledges 20006 can be configured to break-away, i.e., suddenly permit relative movement between the cartridge deck 20010 and the support jaw 20040, when a predetermined force transmitted through the ledges 20006 has been reached or exceeded. Each ledge 20006 of a cartridge deck 20010 can be configured to break-away at the same predetermined force. Alternatives are envisioned in which the ledges 20006 of a cartridge deck 20010 can be configured to break-away at different predetermined forces. In either event, such break-away features can delineate when the staple cartridge 20000 has transitioned between an unfired configuration and an at least partially-fired configuration. Referring to FIGS. 14 and 15 once again, the cartridge deck 20010 has moved toward, or broken-away toward, the bases 20032 of the staples 20030 and the bottom surface 20042 of the support jaw 20040. The cartridge deck 20010 can break-away at the same time that the anvil jaw 20060 starts to deform the staples 20030. Alternatively, the cartridge deck 20010 can break-away at the same time that the staples 20030 are being deformed to their final deformed configurations. The cartridge deck 20010 can be configured to collapse when a sufficient compressive force, or pressure, is applied thereto. The cartridge deck 20010 can comprise a plurality of downwardly-depending supports which can be configured to abut the bottom surface 20042 of the support jaw 20040 wherein such supports can be configured to collapse when a sufficient compressive force, or pressure, is applied to, or transmitted through, the cartridge deck 20010.

Referring to FIGS. 14 and 15 once again, the cartridge deck 20010 can collapse toward the bases 20032 of the staples 20030 and the bottom surface 20042 of the support jaw 20040. For example, the top surface 20042 of the cartridge deck 20010 can move closer to the bases 20031 of the staples 20030 as the cartridge deck 20010 is moved between an unfired position (FIG. 14) and a fired position (FIG. 15). The top surface 20012 of the cartridge deck 20010 is positioned above the top surfaces of the stationary staple drivers 20043 when the cartridge deck 20010 is in an unfired configuration, as illustrated in FIG. 14, and can be positioned below the top surfaces of the stationary staple drivers 20043 when the cartridge deck 20010 is in a fired configuration, as illustrated in FIG. 15. In the fired configuration of the cartridge deck 20010, as a result, the stationary staple drivers 20043 can protrude upwardly from the cartridge deck 20010. For example, the top surface 20012 of the cartridge deck 20010 can be positioned above the bases 20031 of the staples 20030 when the cartridge deck 20010 is in its unfired configuration and, the top surface 20012 can be aligned, or at least substantially aligned, with the bases 20031 of the staples 20030 when the cartridge deck 20010 is in its fired configuration. The bottom surface 20017 of the cartridge deck 20010 can be aligned, or at least substantially aligned, with the bases 20031 of the staples 20030 when the cartridge deck 20010 is in its unfired configuration and, the bottom surface 20017 can be positioned below the bases 20031 when the cartridge deck 20010 is in its fired configuration. In the unfired configuration of the cartridge deck 20010, referring again to FIG. 15, the bottom surface 20017 can be positioned above the bottom surface 20042 of the support jaw 20040, as illustrated in FIG. 14, and can contact the bottom surface 20042 when the cartridge deck 20010 is moved into its fired configuration, as illustrated in FIG. 15. In any event, when the cartridge deck 20010 is moved downwardly toward the bottom surface 20042 of the support jaw 20040, the cartridge deck 20010 can be moved in the direction of the bases 20031 of the staples 20030, for example.

As discussed above, the stationary staple drivers 20043 can be configured to support the staples 20030 along straight, or at least substantially straight, rows, or lines. In such cases, the stationary staple drivers 20043 can also be positioned along straight, or at least substantially straight, rows. For example, the support grooves 20044 defined in the stationary staple drivers 20043 can each be defined along a longitudinal axis which is collinear, or at least substantially collinear, with the longitudinal axes defined by at least some of the other support grooves 20044. The stationary staple drivers 20043 can be configured to support the staples 20030 along at least partially curved rows. In such cases, the stationary staple drivers 20043 can also be positioned along curved rows. For example, the support grooves 20044 defined in the stationary staple drivers 20043 can each be defined along a longitudinal axis which is not collinear with the other longitudinal axes defined by the support grooves 20044 along the curved portion of the staple row.

As outlined above, the staple cartridge 20000 can be positioned on a first side of the targeted tissue and the anvil jaw 20060 can be positioned on a second, or opposite, side of the tissue. In certain circumstances, the distal ends of the support jaw 20040 and the anvil jaw 20060 can be aligned with the targeted tissue and then pushed distally such that the tissue is located between the proximal and distal ends of the staple cartridge 20000. As also outlined above, the tissue thickness compensator 20020 of the staple cartridge 20000 can comprise various tissue-contacting surfaces, such as surface 20021, for example. The tissue thickness compensator 20020 may include any suitable number of bevels, radiused edges, and/or other suitable surfaces, such as lead-in surface 20028, for example, which can facilitate the insertion of the tissue proximal to the tissue thickness compensator 20020 without dislodging the tissue thickness compensator 20020 from the staple cartridge 20000.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

## Claims

1. An end effector for a surgical stapling instrument, comprising:
a staple cartridge (20000), comprising:
a deck (20010) comprising a top surface; and
a plurality of staples (20030) at least partially extending through said deck,
wherein each said staple (20030) comprises a base (20031) and two legs (20032) extending from the base (20031), wherein said deck is slidable relative to said staples (20030) between an unfired position and a fired position;
a support jaw (20040), comprising:
a frame (20042);
a plurality of stationary drivers (20043) extending from said frame (20042) and immovable relative to the support jaw (20040), wherein each said stationary driver (20043) has the base (20031) of a said staple (20030) positioned over its top surface such that the stationary driver (20043) is configured to support the staple (20030) when said deck (20010) is moved into said fired position, and wherein said top surface of said deck (20010) is positioned above said stationary drivers (20043) when said deck (20010) is in said unfired position; and
a well (20046) defined intermediate said stationary drivers (20043), wherein said well (20046) is configured to receive said deck (20010) when said deck (20010) is in said fired position; and
an anvil jaw (20060) movably attached to said support jaw (20040), wherein said anvil jaw (20060) is movable between an unfired position and a fired position, said anvil jaw (20060) comprising:
a tissue-contacting surface (20061); and
a plurality of staple pockets (20063) configured to deform the staples (20030)
wherein said anvil jaw (20060) is configured to drive said deck (20010) between its unfired position and its fired position and to deform said staples (20030) in said staple pockets (20063) as said anvil jaw (20060) is moved between its unfired position and its fired position; and
wherein said deck (20010) further comprises a plurality of apertures (20013) configured to receive said stationary drivers (20043) when said deck (20010) is moved into said fired position,
**characterized in that**
said stationary drivers (20043) are configured to engage the sidewalls of said apertures (20013) in a press-fit manner.

2. The end effector of Claim 1, wherein said stationary drivers (20043) are integral with said frame (20042).

3. The end effector of Claim 2, wherein said frame (20042) and said stationary drivers (20043) are comprised of a unitary piece of metal.

4. The end effector of any preceding Claim, wherein each said stationary driver (20043) comprises a groove (20044) configured to receive a base (20031) of a said staple (20030) therein.

5. The end effector of Claim 4, wherein said support jaw (20040) further comprises a longitudinal slot (20018) configured to receive at least a portion of a cutting member (20053), and wherein said grooves (20044) are parallel to said longitudinal slot (20018).

6. The end effector of Claim 4, wherein said support jaw (20040) further comprises a longitudinal slot (20018) configured to receive at least a portion of a cutting member (20053), and wherein said grooves (20044) are not parallel to said longitudinal slot (20018).

7. The end effector of any preceding Claim, wherein each said stationary driver (20043) comprises an outer perimeter, and wherein said outer perimeter is tapered.

## Patentansprüche

1. Endeffektor für ein chirurgisches Klammernahtinstrument, umfassend:
ein Klammermagazin (20000), umfassend:
eine Plattform (20010), die eine obere Fläche umfasst, und
eine Vielzahl von Klammern (20030), die sich mindestens teilweise durch die Plattform erstrecken,
wobei jede Klammer (20030) eine Basis (20031) und zwei sich von der Basis (20031) erstreckende Schenkel (20032) umfasst, wobei die Plattform bezüglich der Klammern (20030) zwischen einer nicht ausgelösten Position und einer ausgelösten Position verschiebbar ist,
eine Stützbacke (20040), die Folgendes umfasst:
einen Rahmen (20042),
eine Vielzahl von feststehenden Treibern (20043), die sich von dem Rahmen (20042) erstrecken und bezüglich der Stützbacke (20040) unbeweglich sind, wobei die Basis (20031) einer Klammer (20030) bei jedem feststehenden Treiber (20043) über dessen oberer Fläche positioniert ist, so dass der feststehende Treiber (20043) dazu ausgestaltet ist, die Klammer (20030) zu stützen, wenn die Plattform (20010) in die ausgelöste Position bewegt wird, und wobei die obere Fläche der Plattform (20010) über den feststehenden Treibern (20043) positioniert ist, wenn die Plattform (20010) in der nicht ausgelösten Position ist, und
eine Vertiefung (20046), die zwischen den feststehenden Treibern (20043) definiert ist, wobei die Vertiefung (20046) dazu ausgestaltet ist, die Plattform (20010) aufzunehmen, wenn die Plattform (20010) in der ausgelösten Position ist, und
eine Ambossbacke (20060), die beweglich an der Stützbacke (20040) angebracht ist, wobei die Ambossbacke (20060) zwischen einer nicht ausgelösten Position und einer ausgelösten Position beweglich ist, wobei die Ambossbacke (20060) Folgendes umfasst:
eine Gewebekontaktfläche (20061) und
eine Vielzahl von Klammertaschen (20063), die zum Deformieren der Klammern (20030) ausgestaltet sind,
wobei die Ambossbacke (20060) dazu ausgestaltet ist, die Plattform (20010) zwischen ihrer nicht ausgelösten Position und ihrer ausgelösten Position anzutreiben und die Klammern (20030) in den Klammertaschen (20063) zu deformieren, wenn die Ambossbacke (20060) zwischen ihrer nicht ausgelösten Position und ihrer ausgelösten Position bewegt wird, und
wobei die Plattform (20010) ferner eine Vielzahl von Öffnungen (20013) umfasst, die dazu ausgestaltet sind, die feststehenden Treiber (20043) aufzunehmen, wenn die Plattform (20010) in die ausgelöste Position bewegt wird,
**dadurch gekennzeichnet, dass**
die feststehenden Treiber (20043) dazu ausgestaltet sind, die Seitenwände der Öffnungen (20013) mit Presssitz in Eingriff zu nehmen.

2. Endeffektor nach Anspruch 1, wobei die feststehenden Treiber (20043) mit dem Rahmen (20042) integral sind.

3. Endeffektor nach Anspruch 2, wobei der Rahmen (20042) und die feststehenden Treiber (20043) aus einem einstückigen Metallteil bestehen.

4. Endeffektor nach einem der vorhergehenden Ansprüche, wobei jeder feststehende Treiber (20043) eine Nut (20044) umfasst, die zur Aufnahme einer Basis (20031) einer Klammer (20030) darin ausgestaltet ist.

5. Endeffektor nach Anspruch 4, wobei die Stützbacke (20040) ferner einen Längsschlitz (20018) umfasst, der zur Aufnahme mindestens eines Abschnitts eines Schneidglieds (20053) ausgestaltet ist, und wobei die Nuten (20044) parallel zu dem Längsschlitz (20018) verlaufen.

6. Endeffektor nach Anspruch 4, wobei die Stützbacke (20040) ferner einen Längsschlitz (20018) umfasst, der zur Aufnahme mindestens eines Abschnitts eines Schneidglieds (20053) ausgestaltet ist, und wobei die Nuten (20044) nicht parallel zu dem Längsschlitz (20018) verlaufen.

7. Endeffektor nach einem der vorhergehenden Ansprüche, wobei jeder feststehende Treiber (20043) einen äußeren Umfang umfasst und wobei sich der äußere Umfang verjüngt.

## Revendications

1. Effecteur terminal pour un instrument d'agrafage chirurgical, comprenant :
une cartouche (20000) d'agrafes, comprenant :
un pont (20010) comprenant une surface supérieure ; et
une pluralité d'agrafes (20030) s'étendant au moins partiellement à travers ledit pont, dans laquelle chacune desdites agrafes (20030) comprend une base (20031) et deux pattes (20032) s'étendant depuis la base (20031), dans laquelle ledit pont peut coulisser par rapport auxdites agrafes (20030) entre une position non déclenchée et une position déclenchée ;
une mâchoire de support (20040), comprenant :
un cadre (20042) ;
une pluralité de dispositifs d'entraînement fixes (20043) s'étendant à partir dudit cadre (20042) et immobiles par rapport à la mâchoire de support (20040), dans laquelle chaque dit dispositif d'entraînement fixe (20043) présente la base (20031) d'une dite agrafe (20030) positionnée sur sa surface supérieure de telle sorte que le dispositif d'entraînement fixe (20043) soit configuré pour supporter l'agrafe (20030) lorsque ledit pont (20010) est déplacé dans ladite position déclenchée, et dans laquelle ladite surface supérieure dudit pont (20010) est positionnée au-dessus desdits dispositifs d'entraînement fixes (20043) lorsque ledit pont (20010) est dans ladite position non déclenchée ; et
un puits (20046) défini entre lesdits dispositifs d'entraînement fixes (20043), ledit puits (20046) étant configuré pour recevoir ledit pont (20010) lorsque ledit pont (20010) est dans ladite position déclenchée ; et
une mâchoire (20060) d'enclume fixée de manière mobile à ladite mâchoire de support (20040), ladite mâchoire (20060) d'enclume étant mobile entre une position non déclenchée et une position déclenchée, ladite mâchoire (20060) d'enclume comprenant :
une surface de contact avec un tissu (20061) ; et
une pluralité de poches (20063) pour agrafes configurées pour déformer les agrafes (20030)
dans lequel ladite mâchoire (20060) d'enclume est configurée pour entraîner ledit pont (20010) entre sa position non déclenchée et sa position déclenchée et pour déformer lesdites agrafes (20030) dans lesdites poches (20063) d'agrafes à mesure que ladite mâchoire (20060) d'enclume est déplacée entre sa position non déclenchée et sa position déclenchée ; et
dans lequel ledit pont (20010) comprend en outre une pluralité d'ouvertures (20013) configurées pour recevoir lesdits dispositifs d'entraînement fixes (20043) lorsque ledit pont (20010) est déplacé dans ladite position déclenchée,
**caractérisé en ce que**
lesdits dispositifs d'entraînement fixes (20043) sont configurés pour venir en prise avec les parois latérales desdites ouvertures (20013) d'une manière ajustée par pression.

2. Effecteur terminal selon la revendication 1, dans lequel lesdits dispositifs d'entraînement fixes (20043) font partie intégrante dudit cadre (20042).

3. Effecteur terminal selon la revendication 2, dans lequel ledit cadre (20042) et lesdits dispositifs d'entraînement fixes (20043) sont constitués d'une pièce unitaire de métal.

4. Effecteur terminal selon l'une quelconque des revendications précédentes, dans lequel chaque dit dispositif d'entraînement stationnaire (20043) comprend une rainure (20044) configurée pour recevoir une base (20031) d'une dite agrafe (20030) en son sein.

5. Effecteur terminal selon la revendication 4, dans lequel ladite mâchoire de support (20040) comprend en outre une fente longitudinale (20018) configurée pour recevoir au moins une partie d'un élément de coupe (20053), et dans lequel lesdites rainures (20044) sont parallèles à ladite fente longitudinale (20018).

6. Effecteur terminal selon la revendication 4, dans lequel ladite mâchoire de support (20040) comprend en outre une fente longitudinale (20018) configurée pour recevoir au moins une partie d'un élément de coupe (20053), et dans lequel lesdites rainures (20044) ne sont pas parallèles à ladite fente longitudinale (20018).

7. Effecteur terminal selon l'une quelconque des revendications précédentes, dans lequel chaque dit dispositif d'entraînement fixe (20043) comprend un périmètre externe, et dans lequel ledit périmètre externe est conique.
